(19) **Europäisches Patentamt European Patent Office Office européen des brevets**

(11) **EP 4 454 740 A1**

(12) **EUROPEAN PATENT APPLICATION**

(43) Date of publication:
**30.10.2024 Bulletin 2024/44**

(21) Application number: **23170779.5**

(22) Date of filing: **28.04.2023**

(51) International Patent Classification (IPC):
**B01D 69/02** (2006.01)    **B01D 71/02** (2006.01)
**B01D 67/00** (2006.01)    **F03G 7/00** (2006.01)
**G01N 33/487** (2006.01)    **H01M 8/22** (2006.01)

(52) Cooperative Patent Classification (CPC):
**B01D 67/0072; B01D 69/02; B01D 69/105;**
**B01D 71/024; F03G 7/015; G01N 33/48721;**
B01D 2325/0214; B01D 2325/08; H01M 8/227

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ECOLE POLYTECHNIQUE FEDERALE DE LAUSANNE (EPFL)**
**1015 Lausanne (CH)**

(72) Inventors:
• **CHERNEV, Andrey**
  **1022 Chavannes-Renens (CH)**
• **TENG, Yunfei**
  **1024 Ecublens (CH)**
• **RADENOVIC, Aleksandra**
  **1024 Ecublens (CH)**

(74) Representative: **Piticco, Lorena**
  **Isler & Pedrazzini AG**
  **Giesshübelstrasse 45**
  **Postfach 1772**
  **8027 Zürich (CH)**

(54) **NATURE-INSPIRED STALACTITE NANOSTRUCTURES SUCH AS NANOPORES FOR BIOSENSING AND ENERGY HARVESTING**

(57) A method of forming a structure, preferably a pore, comprises the steps of providing a template comprising at least one template aperture, and growing at least one material at an orifice of the template aperture by depositing the material, whereby the structure is formed.

FIG. 1

**(c)**

**(d)**

HfO₂ Stalactites Structure

FIG. 1

## Description

### TECHNICAL FIELD

The present invention relates to a method of forming a structure, preferably a pore, according to claim 1, an assembly comprising a template and a structure, in particular a pore, formed in the said method according to claim 11, an osmotic power generator comprising said assembly according to claim 12, a method of generating osmotic power using said osmotic power generator according to claim 13, a sensing device for sensing an analyte comprising said assembly according to claim 14, and a method of sensing an analyte using said sensing device according to claim 15.

### PRIOR ART

Nature-inspired and artificial nanostructures such as nanopores hold great potential in a wide range of applications, such as biomolecules' sequencing', energy harvesting and generation[2,3], bio-inspired iontronics[4,5], biosensing[6], chemical separation[7,8], and nanofluidics[9]. In particular, there has been growing interest in combining the features of biological pores and channels with the flexibility and robustness of solid-state material membranes that can be fabricated using state-of-the-art technologies[10,11]. The various applications require the pores to be adjustable, ion-selective, scalable, stable, and affordable[6].

Currently, the main problems affecting most of the nanopore applications include limited lifetime and instability of the pores[12], difficulties with wide-range pore-size variation[13], limited ion and molecular selectivity[13], and limited scalability from a single pore to large-scale arrays[14]. Previous experimental and theoretical studies extensively addressed the challenges of nanopore scalability to create the nanoporous arrays[15] and the role of asymmetry that predicted[16] to provide additional benefits for biosensing and power generation[17]. The geometrical asymmetry suggests a gradient of electrical potential along the pore axis, which interferes with the external electric field and creates an electrostatic barrier different for positive and negative ions, allowing ionic rectification and molecular separation by sign, charge, and size. These properties particularly provide good conditions for the application of such pores for energy harvesting, biomolecule analysis, filtration, etc. However, the robust fabrication method of arrays of nanopores with high asymmetry, low resistivity, and scalability remains elusive.

### SUMMARY OF THE INVENTION

It is an object of the present invention to provide a robust method that allows the formation of structures, in particular of pores having improved properties for energy harvesting, biomolecule analysis, filtration, etc.

This object is achieved with a method according to claim 1. That is, a method of forming a structure, preferably a pore, is provided. The method comprises the steps of i) providing a template comprising at least one template aperture, and ii) growing at least one material at an orifice of the template aperture by depositing the material, whereby the structure is formed.

That is, the present invention is based on the insight that structures such as pores can be grown and thus formed by depositing at least one material the structure such as the pore shall comprise or consist of at an orifice of a template aperture of a template.

That is, a preferred structure according to the invention is a pore. The pore preferably comprises a pore opening and thus preferably is a hollow structure. However, it is likewise conceivable that the structure does not comprise any such opening or is not hollow, respectively. That is, the structure can be a solid structure.

As will be explained in greater detail below, the deposition of the material resulting in the growth and thus formation of the structure such as the pore preferably occurs in a tailored Atomic Layer Deposition process and particularly preferably in conditions of anisotropic growth. In particular, the structure such as the pore is formed by depositing the material via said tailored Atomic Layer Deposition (ALD) process, wherein the structure such as the pore is grown in a stepwise manner i.e. in multiple growth cycles. Herein, said tailored Atomic Layer Deposition process is referred to as a non-ALD process. The resulting structure such as the pore is asymmetric, wherein its aspect ratio is determined by the number of growth cycles, with a final structure such as a final pore size being preferably smaller than the initial size of the orifice of the template aperture. In particular, structures such as pores of a size in the nanometer range can be formed, i.e. the method of the invention enables the formation of nanostructures such as nanopores. However, structures such as pores of another size such as in the micrometer range can likewise be formed, i.e. the method of the invention enables the formation of microstructures such as micropores. In fact, the method of the invention allows the formation of structures such as pores of a desired size, wherein the size can be determined by the number of growth cycles and/or the initial size of the orifice of the template aperture.

The material is preferably also deposited and grown on regions of the template other than the region comprising the orifice of the template aperture, see further below. Said deposition preferably occurs in an ALD process as it is well known in the art, herein called standard ALD process. In standard ALD, a linear deposition and thus isotropic growth of the material occurs. Said linear deposition or isotropic growth preferably results in the formation of a layer. This is in clear contrast to the non-ALD process of the present invention, wherein an anisotropic growth of the material occurs thus allowing the formation of the structure such as the pore. Hence, the growth rate of the material associated with the non-ALD process preferably differs from the growth rate of the material associated with the standard ALD process. In particular, the growth rate of the material at the orifice of the template aperture being grown in the non-ALD process is larger, preferably by at least one and more preferably by at least two orders of magnitude, than the growth rate of the material in template aperture-free regions of the template being grown in the standard ALD. As a result, a structure such as a pore is grown and thus formed at the orifice of the template aperture.

The material is preferably grown by supplying at least two precursors in two or more supply cycles. These precursors are preferably supplied into a reaction chamber. The template is preferably arranged in the said reaction chamber as well. In a first supply cycle, the precursors preferably react with the orifice of the template aperture, whereby the material is deposited at the orifice of the template aperture. In subsequent supply cycles, the precursors preferably react with the material being deposited in a previous supply cycle, whereby the deposited material grows in growth cycles and preferably in the form of rings.

That is, the template comprising the template aperture is preferably placed into a reaction chamber. Said reaction chamber preferably corresponds to a reaction chamber as it is commonly used in standard ALD. Thereafter, at least two precursors are preferably alternatingly supplied into the reaction chamber, preferably as pulses.

Various precursors are conceivable. Said precursors are preferably well-known precursors in the field of standard ALD.

For example, a first precursor comprising at least one metal compound and/or non-metal compound and a second precursor comprising at least one compound reacting with the first precursor could be used. A conceivable first precursor comprising at least one metal compound is a first precursor comprising at least one organometal compound such as tetrakis(ethylmethylamido)hafnium. Another example is a first precursor comprising at least one inert metal such as platinum. Another example is an inorganic compound such as titanium tetrachloride ($TiCl_4$). A conceivable first precursor comprising at least one non-metal compound is a first precursor comprising at least one silicon compound. Conceivable second precursors comprise at least one oxidant such as water vapor, oxygen ($O_2$), ozone (Os), nitrogen ($N_2$), ammonia, and a plasma such as an oxygen plasma or formation-assistant chemical. Formation-assistant chemicals are well-known in the art and are preferably used for inert metal deposition such as platinum deposition.

Hence, the deposited material and as such the structure such as the pore preferably comprises or consists of at least one non-metal compound and/or metal compound and/or a metal. For instance, the deposited material preferably comprises or consists of at least one metal compound such as a metal oxide, for instance, hafnium oxide ($HfO_2$), or a metal nitride, for instance, titanium nitride (TiN). The deposited material could likewise comprise or consist of a metal such as an inert metal, for instance, platinum (Pt), or a non-metal compound such as silicon dioxide ($SiO_2$). It is noted that this list is not exhaustive. Instead, the deposited material and as such the structure such as the pore can consist of a variety of other compounds, depending on which first and second precursors are used.

The precursors, in particular the first precursor and the second precursor, are preferably provided as alternating precursor pulses, wherein in each of these precursor pulses the precursors react with the orifice of the template aperture or with the material being already deposited in a self-limiting way. The precursor pulses are preferably non-overlapping with respect to time.

The pulse duration of the precursor pulse supplying the first precursor and the pulse duration of the precursor pulse supplying the second precursor are preferably different from one another.

The particular pulse durations are preferably chosen in accordance with the type of precursors, i.e. their compounds. For instance, in the event of the first precursor being an organometal compound such as tetrakis(ethylmethylamido)hafnium and the second precursor being water vapor a conceivable pulse durations are in the range of 1 second to 3 seconds such as about 2 seconds.

Furthermore, temporal pauses can be present between two successive pulses. For instance, a temporal pause of 10 seconds or less, preferably of 8 seconds or less such as 5 seconds or less or 2 seconds or less, could be present between the first precursor supplying the organometal compound such as tetrakis(ethylmethylamido)hafnium, and the second precursor supplying the water vapor.

As mentioned initially, the deposition of the material via this tailored Atomic Layer Deposition (ALD) process results in a structure such as a pore being grown in different supply cycles, i.e. in multiple growth cycles and in a stepwise manner. Each supply cycle deposits new material on material that has been deposited in the previous supply cycle. The previously deposited material thus forms a template for the material to be deposited.

The shape of the orifice of the aperture preferably determines the shape of the deposited material. For instance, if the orifice of the template aperture is of a circular shape, the deposited material preferably has, when seen in cross-section, a circular shape as well. The material being deposited per supply cycle or that grows per growth cycle can thus be said to have the form of a ring. Hence, when growing, the deposited material such as the rings of deposited material grows along a structure axis such as a pore axis extending from a base, e.g. the pore base, towards a tip, e.g. the pore tip, wherein previously deposited material such as a preceding ring of deposited material is a template for the further material such as the next ring of material to be deposited.

Hence, the structure such as the pore preferably comprises the region of the orifice of the template aperture a structure base such as a pore base. Moreover, the structure such as the pore preferably extends from the orifice of the template aperture along the structure axis such as the pore axis and terminates in a structure tip such as a pore tip.

It is furthermore preferred that the structure such as the pore grows along the structure axis such as the pore axis with a gradually reducing diameter. In other words, the method according to the invention allows the formation of structures such as pores whose size varies, and wherein the diameter of the structure such as the pore preferably decreases with respect to the structure axis such as the pore axis.

The size of the structure base such as the pore base is preferably determined by the size of the orifice of the template aperture, in particular of the initial orifice prior to any deposition of the material.

The size of the structure tip such as the pore tip is preferably smaller than the size of the orifice of the template aperture

As mentioned initially, the method according to the invention allows the formation of structures such as pores that are hollow, wherein the deposited material such as the said rings of deposited material delimits a hollow space. In other words, the structure preferably has an opening such as the pore having a pore opening. However, and as mentioned initially, it is likewise conceivable that the material is deposited until a non-hollow structure that does not comprise any opening or the like is grown.

The length of the structure is preferably determined by the number of supply cycles. Additionally or alternatively, the structure preferably extends from the orifice of the template aperture along a structure axis and terminates in a structure tip, the size of the structure tip being determined by the number of supply cycles.

That is, the size of the structure tip such as the pore tip depends on the number of supply cycles and can be as low as 3 nanometers.

Hence, the method according to the invention provides several ways of determining the size of the structure such as the pore. Namely, the size of the structure such as the pore can be determined by the number of growth cycles or supply cycles. Furthermore, the size of the structure such as the pore can be determined by the size of the orifice of the template aperture, wherein a decrease in the orifice of the template aperture results in smaller structures such as pores for the same number of growth cycles.

The structure preferably is of an asymmetric shape and/or a conical shape.

That is, the structure such as the pore preferably has an asymmetric shape such as a conical shape, i.e. the size of the structure tip such as the pore tip is different from the size of the structure base such as the pore base, in particular, a wide structure base such as a pore base and a narrow structure tip such as pore tip. This is in clear contrast to a symmetric structure such as a pore that is drilled, for instance in the shape of a cylinder (RIE drilled) or that has an hourglass shape (TEM drilled).

The template preferably is a suspended membrane arranged on a substrate comprising at least one opening. The suspended membrane preferably spans the opening of the substrate. The material to be deposited is preferably supplied to the suspended membrane and enters the opening of the substrate via the suspended membrane. The material is preferably growing inside the opening of the substrate.

The template or membrane preferably comprises or consists of silicon nitride (SiNx) such as SiN. However, many other compounds are conceivable that is suited for forming a thin membrane and that be free-standing (low stress) and allow the formation of the template apertures.

The thickness of the template or membrane, respectively, preferably is in the nanometer range, for instance between 5 nanometers to 50 nanometers such as about 20 nanometers.

The substrate preferably comprises or consists of at least one of SiNx, SiO2, or Si.

The membrane and/or the substrate are preferably commercially available and/or well-known in the art. For instance, the substrate can be manufactured from a template wafer, for instance, a silicon template wafer, wherein one or more layers such as silicon dioxide (SiO2) layers are formed on the template wafer through conventional microfabrication techniques. It is noted that structurally, $SiO_2$ could be omitted. Instead, a template wafer such as a silicon wafer Si in which the opening is formed and a template such as SiNx used as a free-standing membrane that closes the opening would suffice. However, $SiO_2$ can be added to improve electrical characteristics.

It is furthermore preferred that the template, in particular the membrane, is formed on the template wafer or, if applicable, on one of the layers such as the silicon dioxide layer being formed on the template wafer through conventional microfabrication techniques as well.

Thereafter, it is preferred to form the opening in the substrate, for instance by drilling such as TEM drilling or RIE drilling, focused ion beam, or other means as it is well known in the art as well.

The template aperture of the template is preferably arranged congruently with the opening of the substrate. That is, the template aperture preferably leads or opens into the opening of the substrate.

As mentioned earlier, the material is preferably deposited via a non-ALD process. To this end, it is preferred to arrange the substrate in the reaction chamber such, that the material to be deposited or the precursors, respectively, are supplied to the suspended membrane that spans the opening of the substrate. The material or the precursors then enter the opening of the substrate via the suspended membrane, in particular via the at least one template aperture. As such, the material or precursors get trapped inside the opening of the substrate. To this end it is particularly preferred that the first precursor is already trapped inside the opening of the substrate when the second precursor is entering the opening of the substrate.

The template in the form of a suspended membrane is preferably arranged on the first side or top side of the substrate. The opening of the substrate preferably extends at least partially or entirely through the substrate towards an opposed second side or bottom side of the substrate.

The substrate comprising the template in the form of the suspended membrane being formed thereon can be seen as a chip, wherein the opening of the substrate provides an opening of the chip. Hence, the chip is preferably arranged in the reaction chamber such, that the material to be deposited or the precursors, respectively, are supplied to the template, and wherein the material to be deposited or the precursors, respectively, that arrive at the template can flow or drop through the template opening of the template into the opening of the chip, where it precipitates and forms the structure such as the pore. Since the structure such as the pore is formed by flowing or dropping material, the structure such as the pore can also be referred to as a stalactite structure, in particular a stalactite pore.

In this arrangement of the chip, the opening of the chip being spanned by the template preferably delimits the opening of the chip towards an outside. Consequently, the opening of the chip preferably forms a compartment delimited by the template and by walls of the chip delimiting the opening of the chip.

It has turned out that this arrangement results in a very accurate and heterogeneous precursor distribution within the compartment and in the vicinity of the template aperture, such that precipitation and thus deposition of the material takes place inside the opening of the chip and at the orifice of the template aperture. In particular, due to the heterogeneity of the chip caused by the presence of the template aperture(s) and presumably furthermore due to local concentration gradients of the precursors, precipitation is mainly at the orifice of the template aperture(s), wherein the structure such as the pore is growing and thus forming at the orifice of the template aperture.

In particular, the template aperture(s) can be seen as tiny "windows" in the template- once the precursors get inside the compartment of the chip- they are trapped and cannot leave like they could do on a regular surface, for instance. Hence, when a subsequent precursor is supplied there is an excess of the previously supplied precursor present in these "windows". As a consequence, the precursors are trapped within the compartment of the chip and thus prevented from diffusing away.

This is in contrast to a standard ALD that can occur in regions of the template that are aperture-free, wherein the absence of the template apertures and possibly further the absence of an opening in the substrate and ultimately an absence of the before-mentioned compartments prevents trapping of the material to be deposited or the precursors, respectively. As a consequence, a linear deposition or isotropic growth occurs.

The material is preferably furthermore deposited on the template in aperture-free regions of the template, wherein the deposited material preferably forms a layer on the template.

That is, it is preferred to deposit the material also in aperture-free regions of the template. Said deposition is preferably performed in standard ALD.

Hence, the method according to the invention enables a standard ALD growth in aperture-free regions and a selective, anisotropic growth at the orifice of the template aperture.

The template aperture is preferably pre-patterned by lithography, in particular by electron-beam lithography, photolithography, deep UV lithography (DUV), or extreme UV lithography (EUV). Additionally or alternatively, the template aperture is preferably formed by etching, in particular dry etching.

The template aperture is particularly preferably formed by pre-patterning with lithography followed by dry etching.

Said template aperture is preferably formed in the template after the template has been arranged on the substrate. In particular, the template aperture is preferably formed in the chip mentioned earlier.

The template can comprise a single template aperture. Alternatively, the template can comprise a plurality of template apertures.

The plurality of template apertures is preferably arranged in an array. The size of the orifices of the template apertures can be the same or different from one another. In particular, the orifices of the template apertures can comprise gradient sizes.

The size of the orifice preferably is in the nanometer range. Additionally or alternatively, the size of the orifice preferably is between 1 nanometer to several micrometers.

A distance between successive template apertures can be in the nanometer range, such as between 10 nanometers to 500 nanometers, for example between 250 nanometers and 300 nanometers. However, other distances are likewise conceivable. In fact, the distance between the template apertures has been shown not to affect the growth of the deposited material. As such, any distance between template apertures can be used as long as the template apertures are separated from one another.

The surface property of the structure such as the pore is preferably modified by depositing at least one surface modifier on the surface of the structure such as the surface of the pore. To this end the surface modifier can be a chemical surface modifier, i.e. the surface property of the structure such as the pore can be modified chemically, for instance via silanization. Said modification allows, for instance, the formation of positively or negatively charged surfaces.

Other modifications of the surface property are however likewise conceivable and well-known in the art as well. For example, a surface modifier in the form of a thin film technologycompatible material could be deposited on the surface of the structure such as on the surface of the pore.

Additionally or alternatively, the surface property of the structure such as the pore could likewise be modified structurally, in particular shrank, for instance by depositing a surface modifier, and wherein said surface modifier preferably corresponds to one or more precursors known in the field of standard ALD. For example, the structure such as the pore could be grown and formed according to the method so as to have the size of the structure tip such as the pore tip to be around 10 nm. Thereafter, the surface property of the structure such as the pore could be modified structurally by depositing a material like $SiO_2$ in order to slowly shrink the initial structure such as the initial pore to a desired size of the structure tip such as the pore tip, for example, 1 nanometer or even in the sub-nanometer range.

A least one pore formed in the method as produced in the method described above is preferably used for osmotic power generation, sensing, or filtration.

In another aspect an assembly comprising or consisting of a template and at least one structure, in particular at least one pore, formed in the method described above is provided.

Any statements made herein regarding the method of forming the structure such as the pore preferably likewise apply to the assembly comprising the structure such as the pore and vice versa.

The assembly can comprise exactly one structure such as exactly one pore or two or more structures such as two or more pores, for instance, a plurality of structures such as a plurality of pores.

The template preferably corresponds to the template described above. That is, the template preferably comprises at least one template aperture, and wherein the structure such as the pore is grown at an orifice of the template aperture. The template preferably is a membrane, in particular a free-standing membrane. The template or membrane preferably comprises or consists of silicon nitride (SiNx) such as SiN. The thickness of the template or membrane, respectively, preferably is in the nanometer range, for instance between 5 nanometers to 50 nanometers such as about 20 nanometers. Etc.

The assembly is preferably configured for osmotic power generation or sensing, see further below.

However, as mentioned above, the structure such as the pore according to the invention can be used for other applications as well, for instance for filtration. To this end, the structure such as the pore allows one to discriminate species by their size. In particular, species larger than the structure opening such as the pore opening are filtered out.

As such, the assembly according to the invention can be configured for filtration.

In particular, a filtration device for filtering species is provided, wherein the filtration device comprises or consists of at least one assembly as described above, and wherein the structure such as a pore is configured to filter out species being larger than the size of the pore tip but smaller than the size of the pore base.

As mentioned earlier, the structure according to the invention must not in any case be hollow or comprise an opening, respectively. As such, further applications of the structure according to the invention are conceivable. For instance, a plurality of such non-hollow structures could be used as x-ray antennas in the nanometer range, for instance.

In another aspect, an osmotic power generator is provided. The osmotic power generator comprises at least one active membrane, at least a first compartment being disposed on the first side of the active membrane and being configured to receive a first electrolyte and a second compartment being disposed on a second side of the active membrane and being configured to receive a second electrolyte. The first electrolyte has greater ionic strength than the second electrolyte. The active membrane comprises or consists of the assembly as described above. The structure is a pore, and wherein the pore is configured to allow ions of the first electrolyte to pass from the first compartment to the second compartment by diffusion, whereby osmotic power can be generated.

That is, the assembly comprising or consisting of a template comprising at least one structure being at least one pore according to the invention can be used as an active membrane in an osmotic power generator.

In fact, the pore according to the invention has shown to outperform existing osmotic power generators being based on pore platforms, wherein the pore according to the invention is orders of magnitude more stable and is furthermore capable of generating the same power at a more normal pH-value, in particular at a pH-value of 9 instead of 11, for instance.

To this end, it is preferred that the first compartment of the osmotic power generator comprises a first electrolyte such as high salinity water, and that the second compartment of the osmotic power generator comprises a second electrolyte such as low salinity water. These compartments are separated by the active membrane. The osmotic gradient, here the salinity gradient, between the two compartments, leads to a diffusion.

It is furthermore preferred to flush the first and second compartments in order to keep an osmotic gradient constant. To this end it is preferred to flush the first and second compartments with the first and second electrolytes, respectively, in order to keep the same concentrations in the high- and low-salt compartments as it was initially the case.

In the event that a surface property of the pore has been modified so as to be of a particular charge, the pore allows the passing of specific species such as ionic species of a given sign only. As a result, selectivity of the active membrane is provided that can limit the diffusion so that only ionic species of the particular sign, for instance, cations, can pass. Ultimately, this results in species of a net negative sign on one side of the active membrane and species of a net positive sign on the other side of the active membrane.

The osmotic power being produced can be harvested, for example by providing at least a first electrode in electrical connection with the first compartment and at least a second electrode in electrical connection with the second compartment, and wherein the electrodes are configured to harvest the electricity being produced by the diffusion.

In another aspect, a method of generating osmotic power is provided. The method comprises the steps of i) providing at least one osmotic power generator as described above and ii) supplying the first electrolyte into the first compartment and supplying the second electrolyte into the second compartment. The first electrolyte has greater ionic strength than the second electrolyte, wherein ions of the first electrolyte pass through the pore from the first compartment to the second compartment by diffusion, whereby osmotic power is generated.

The assembly according to the invention is preferably arranged such, that an osmotic gradient between the first and second electrolytes establishes along a tip-to-base flow direction.

The osmotic gradient corresponds to a ratio between an ion concentration of the first electrolyte with respect to an ion concentration of the second electrolyte.

The tip-to-base flow direction is understood as the ions passing through the pore from the tip of the pore toward the base of the pore.

In another aspect, a sensing device for sensing an analyte is provided. The sensing device comprises at least one active membrane, at least a first compartment being disposed on the first side of the active membrane and being configured to receive a first electrolyte comprising the analyte, at least a second compartment being disposed on a second side of the active membrane and being configured to receive a second electrolyte, and at least a first electrode being in electrical connection with the first compartment and a second electrode being in electrical connection with the second compartment. The active membrane comprises or consists of the assembly as described above. The structure is a pore, and wherein the pore is configured to allow ions of the first and second electrolyte to pass between the first compartment and the second compartment upon the application of a potential difference between the first electrode and the second electrode, whereby an ionic current is changeable by a translocation of the analyte through the pore.

The active membrane preferably comprises a single pore only, i.e. the assembly according to the invention preferably comprises the template and a single pore formed in the method according to the invention.

The ionic strength of the first electrolyte and the second electrolyte can be the same or different from one another.

The analyte preferably is a molecular analyte. Furthermore, the analyte is preferably charged. For instance, the analyte could be DNA.

That is, the assembly according to the invention can be used for molecular sensing, in particular biosensing.

Upon the application of a potential difference between the first and second electrodes (an ionic current is generated by the ions of the first and second electrolytes passing through the pore between the first compartment and the second compartment. When the analyte translocates or passes through the pore it changes the ionic current. In particular, in the event that the analyte is passing through the pore, it preferably causes a drop in the current level of the ionic current as the resistance increases. By reading out the changes in the ionic current the analyte can be sensed.

In another aspect, a method of sensing an analyte is provided. The method comprises the steps of i) providing at least one sensing device as described above, ii) supplying the first electrolyte comprising or consisting of the analyte into the first compartment, iii) supplying the second electrolyte into the second compartment, and iv) applying a potential difference between the first electrode and the second electrode, wherein ions of the first and second electrolyte pass through the pore between the first compartment and the second compartment, whereby an ionic current is changed by a translocation of the analyte through the pore.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

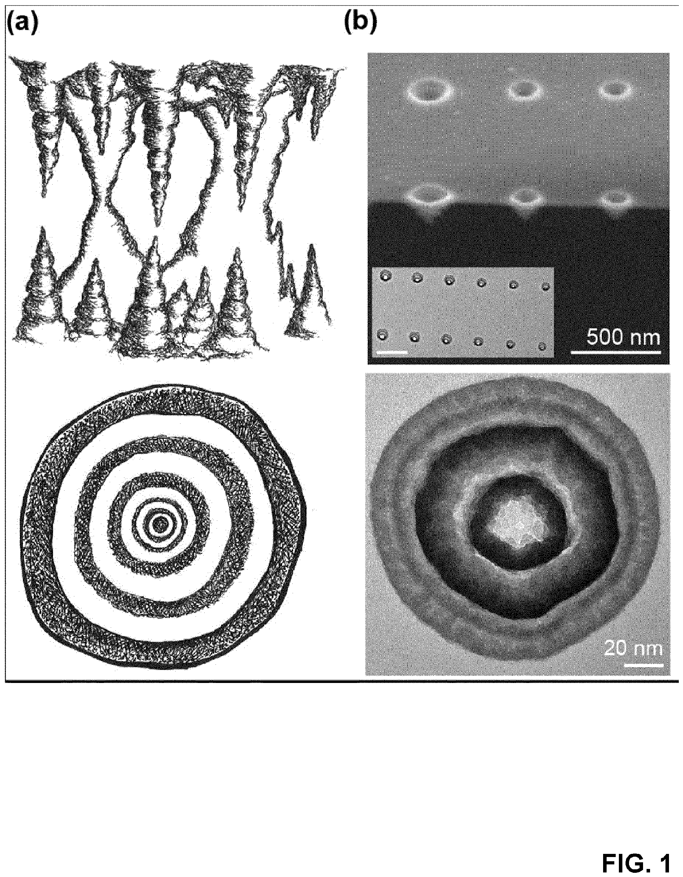

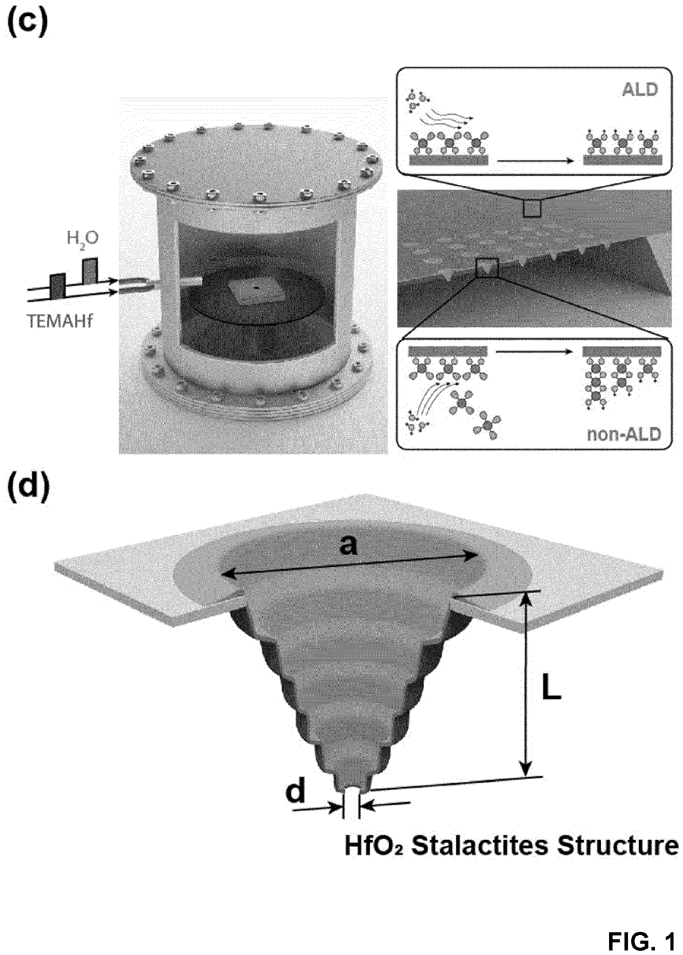

Fig. 1 shows natural and nature-inspired stalactite nanostructures. (a) Stalactites and stalagmites (top): the natural formations created by using periodic thermodynamic conditions and material supply, and the stalactite cross-section schematic (bottom): the rings correspond to the growth cycles. (b) Electronic microscopy (EM) micrographs of HfO2 stalactite nanopores on the silicon-nitride template apertures obtained in the method according to the invention: the panoramic scanning electron microscope (SEM) image (top), and the high-resolution transmission electron microscopy (HR-TEM) top view of a single pore (bottom) with similar to the periodic stalactite structure. The inset shows the HRTEM of several nanopores. Scale bar, 500 nm. (c) The growth procedure consists of the standard ALD and the selective template aperture growth according to the invention. (d) Schematic 3D view of the stalactite nanopore. Arrows show variable dimensions of the nanopore;

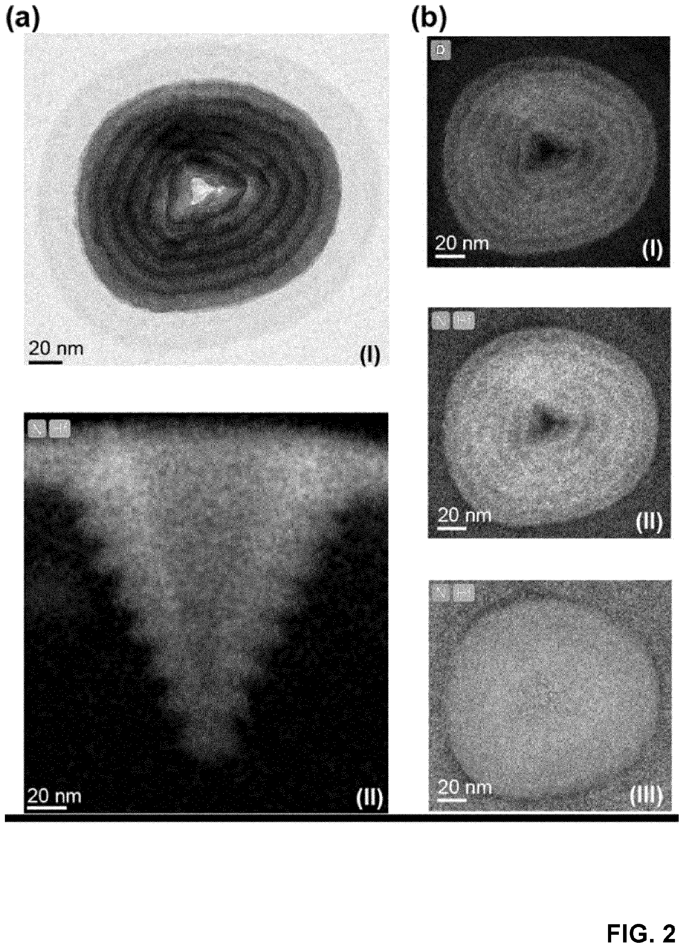

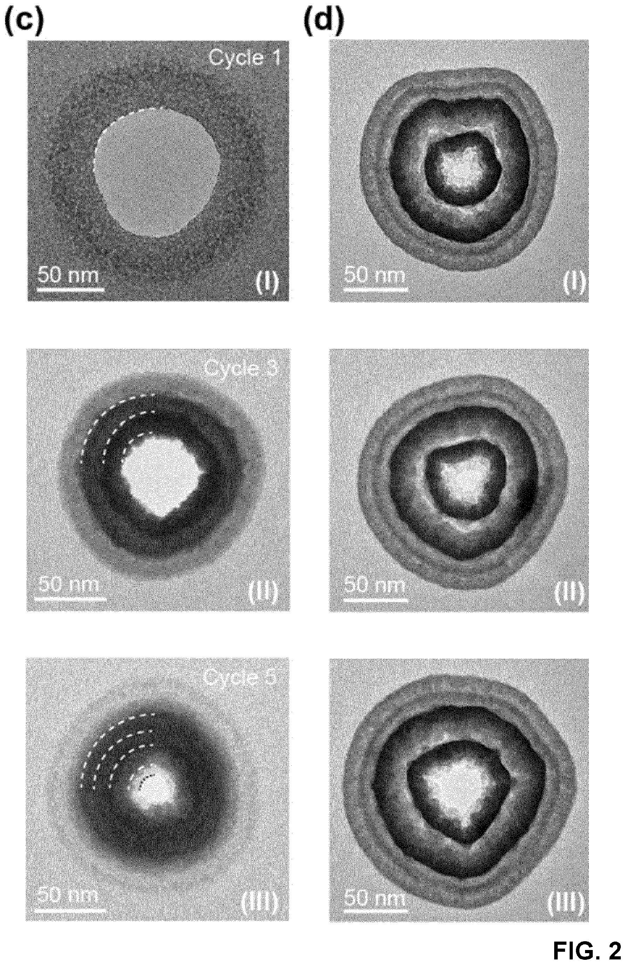

Fig. 2 shows transmission electron microscope (TEM) imaging of single stalactite nanopores. (a) High-resolution TEM image (I). The corresponding energy-dispersive X-ray (EDX) mapping (II). hafnium atoms signals are localized at the stalactite structure, and nitride atoms signals are localized at the top-flat part of the stalactite structure (the silicon nitride membrane). (b) Scanning transmission electron microscopy (STEM) image (I). The corresponding EDX mapping in the absolute (II) and renormalized by thickness (III) values. (c) The pore after one (I), three (II), and five (III) growth cycles. The dashed curves are guides for the eye. (d) Electronic microscopy (EM) micrographs of the pore with the initial aperture diameter of 100 (I), 120 (II), and 150 (III) nm;

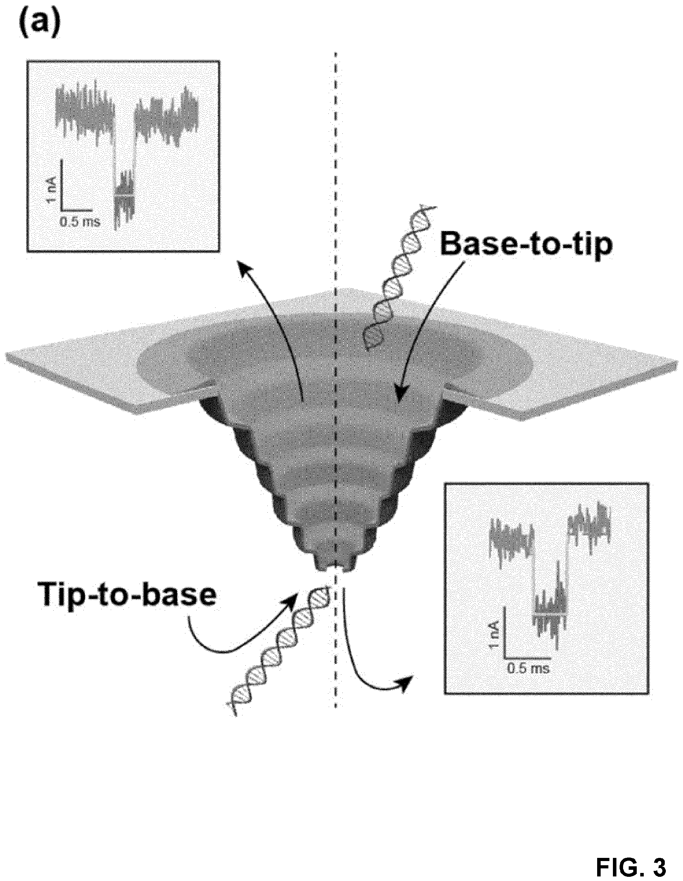

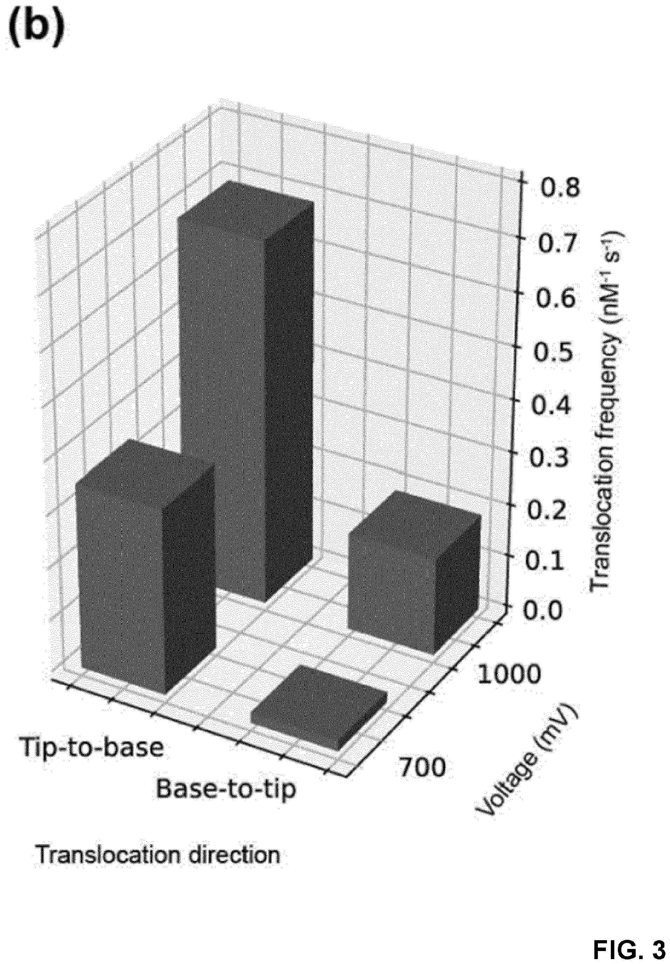

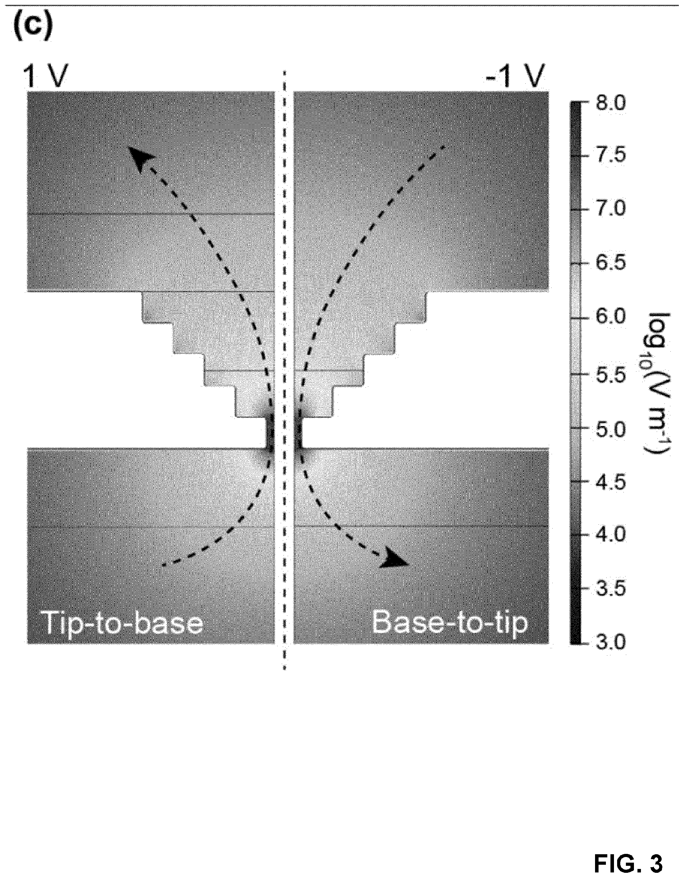

Fig. 3 shows single stalactite nanopore as a sensing device in the form of a biosensor. (a) Experimental layout for tip-to-base and base-to-tip directions for DNA translocation. Insets show the current trace of the translocation events. (b) Concentration-normalized DNA translocation rate in two directions as a function of applied voltage. (c) Nonuniform electric field distribution inside the pore (log scale). Arrows show the direction of DNA translocation in two principal directions;

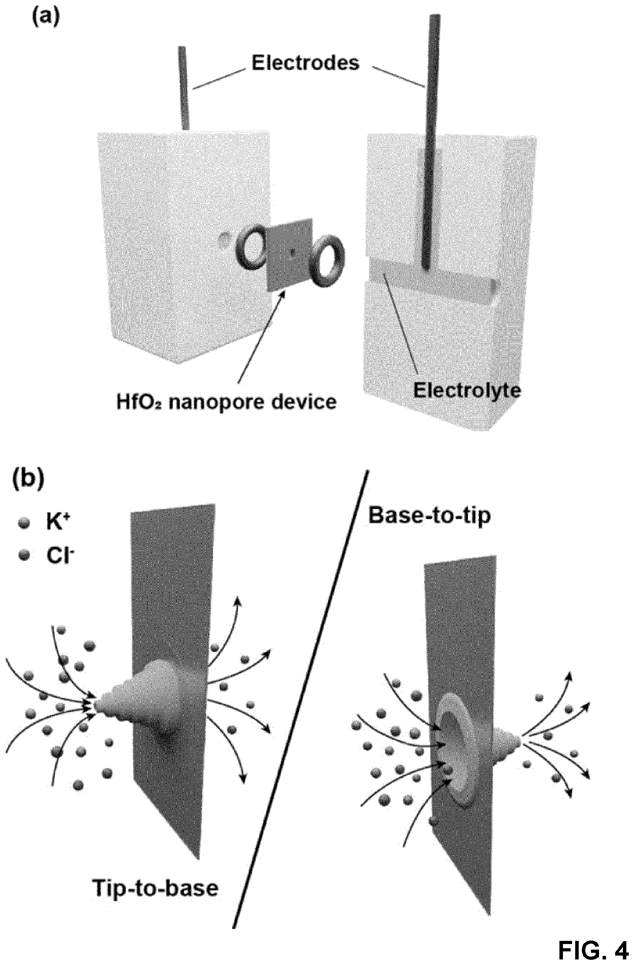

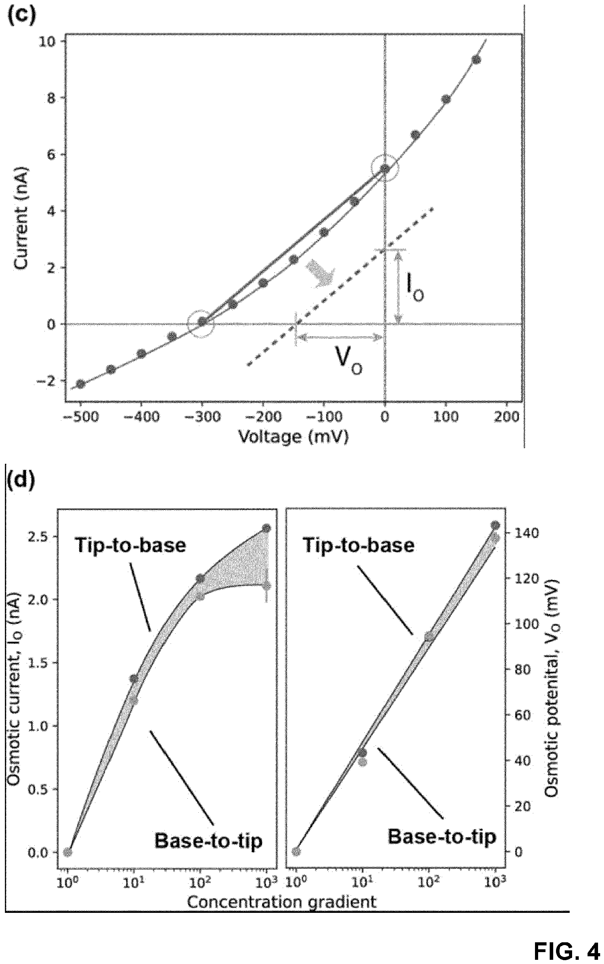

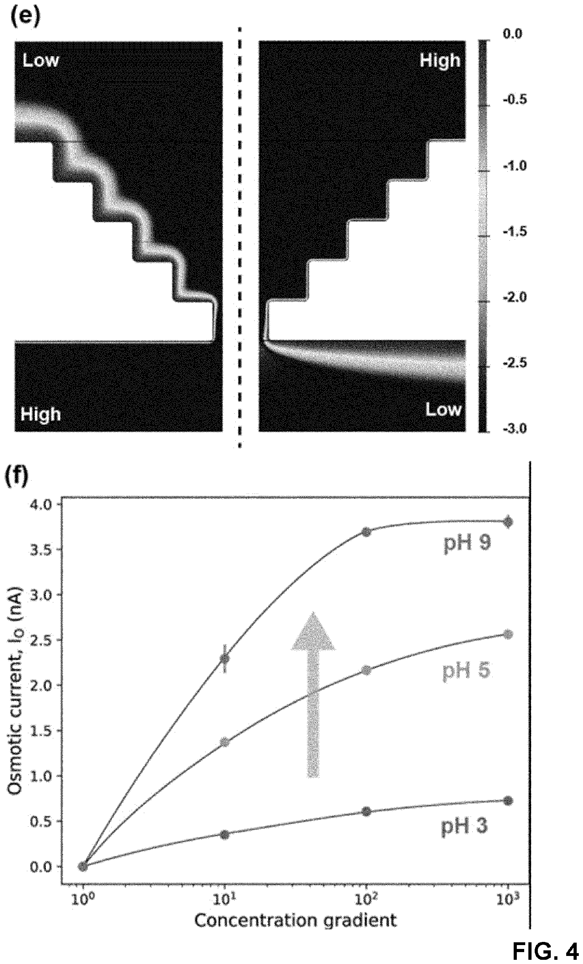

Fig. 4 shows reversed electrodialysis (RED) experiment on a single stalactite nanopore. (a) Setup layout. (b) 3D reconstruction of a single nanopore. Arrows show the ionic current for tip-to-base (left) and base-to-tip (right) directions. The left side has a higher ion concentration compared to the right side. (c) The current-voltage characteristics of the stalactite nanopore for the tip-to-base direction of the 1000-fold concentration gradient at around pH=5 condition. Lines are guides for the eye. Vo and Io are osmotic voltage and osmotic current, respectively, calculated according to previous publication[28]. (d) Osmotic current (left) and osmotic voltage (right) at different salt concentration gradients at pH = 5. The gradient is given as a ratio between the concentrations on the opposite sides of the membrane. (e) Electric charge distribution in the case of tip-to-base (left) and base-to-tip (right) directions of the concentration gradient. (f) Osmotic current vs. concentration gradient at different pH for the tip-to-base gradient;

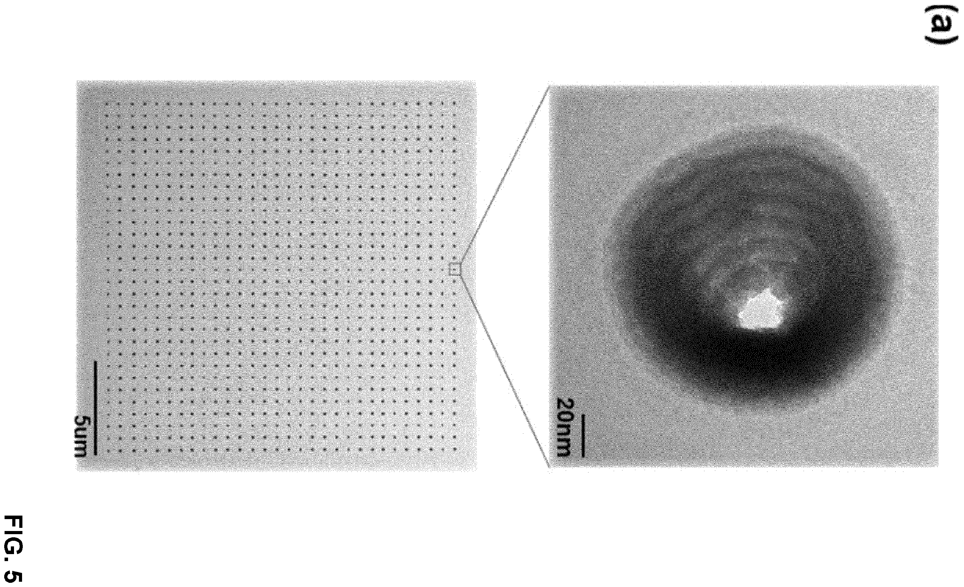

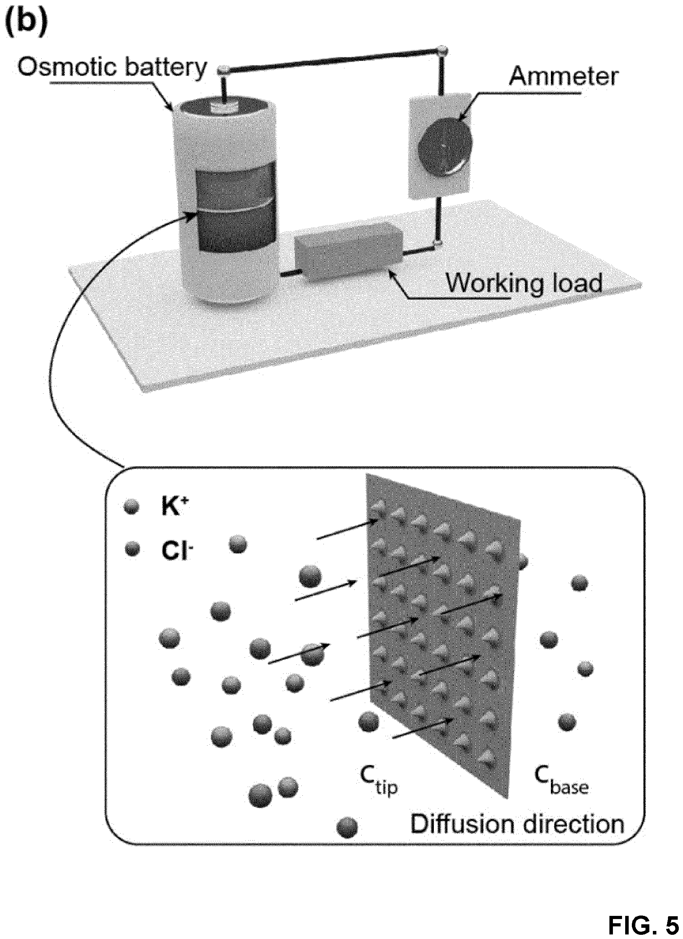

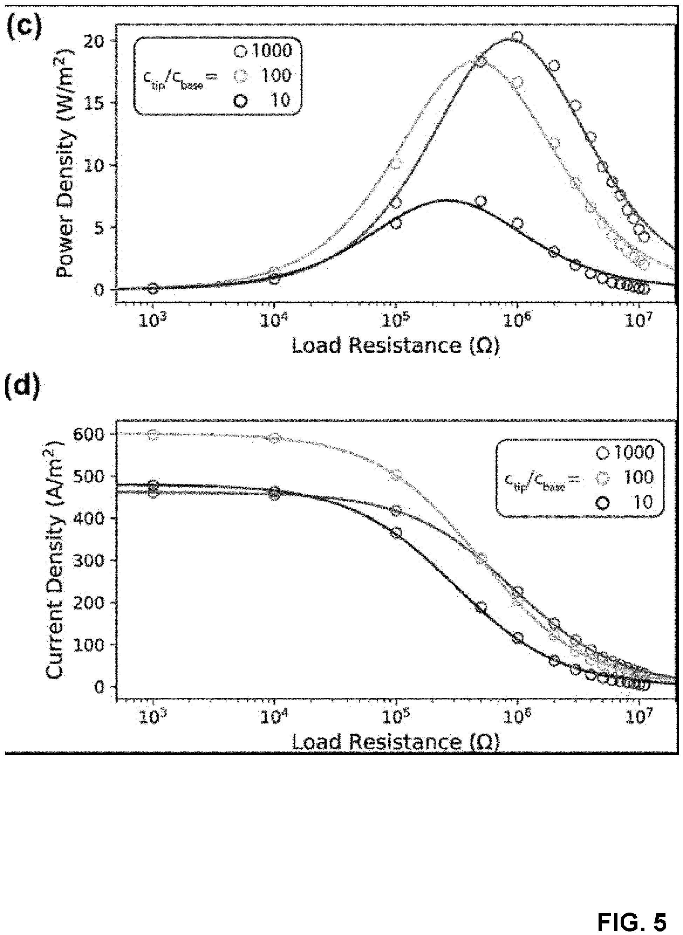

Fig. 5 shows osmotic power generation with an array of stalactite nanopores. (a) Single $HfO_2$ sta-

lactite nanopores conical nanopore (HR-TEM image, top) out of an array of 30 x 30 = 900 nanopores (TEM image, bottom) on a 400 $\mu m^2$ surface of a freestanding silicon nitride membrane. The pores have a base of 100±5 nm and a tip of 15±5 nm (b) Osmotic power generation setup layout (top), and 3D representation of the membrane (bottom). Arrows show the direction of the current flow from high to low salt concentration. (c) and (d) Measured power and current density, respectively, for the tip-to-base direction of the concentration gradient. Three curves are for the concentration of 0.1 M, 0.01 M, and 0.001 M on the low-concentration side. The concentration of the high-concentration side is fixed and equal to 1 M. All the curves are measured at around pH = 5;

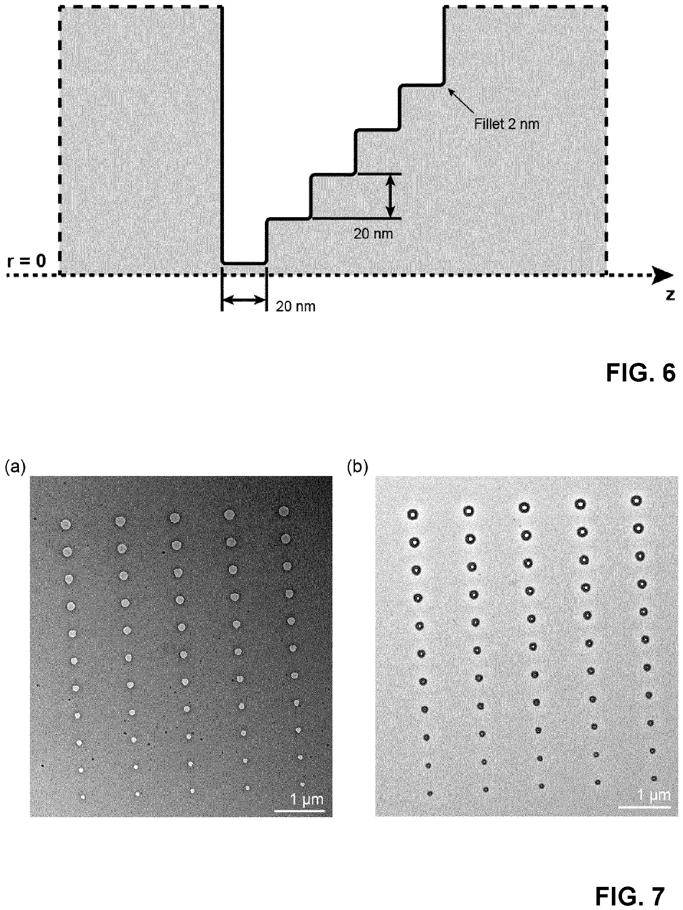

Fig. 6 shows a finite element simulation;

Fig. 7 shows TEM image of stalactite $HfO_2$ structure on Silicon Nitride template aperture with increasing diameters. (a) Silicon nitride template apertures patterned by E-beam lithography and dry etching. (b) Stalactites $HfO_2$ nanostructures grow on silicon nitride apertures;

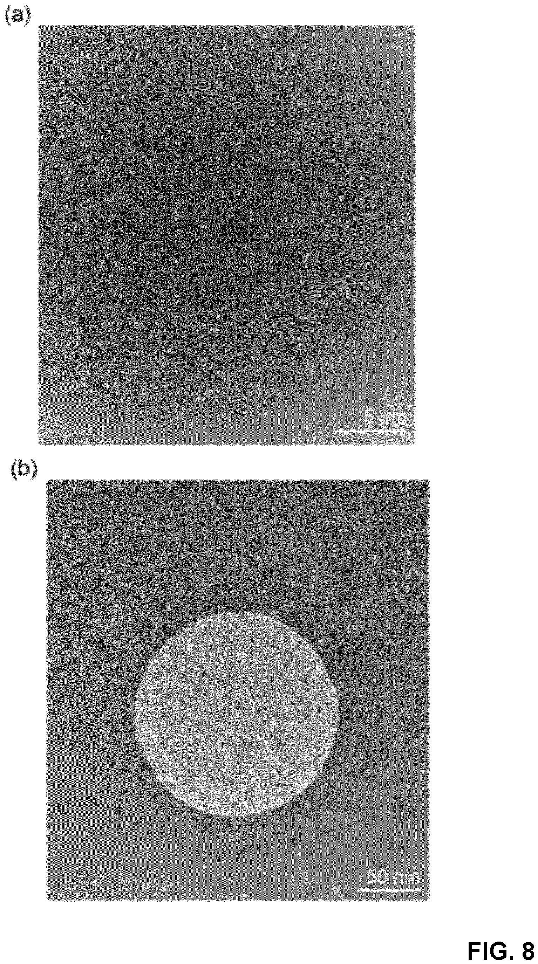

Fig. 8 shows TEM images of silicon nitride template apertures: (a) 30 by 30 array and (b) single circular;

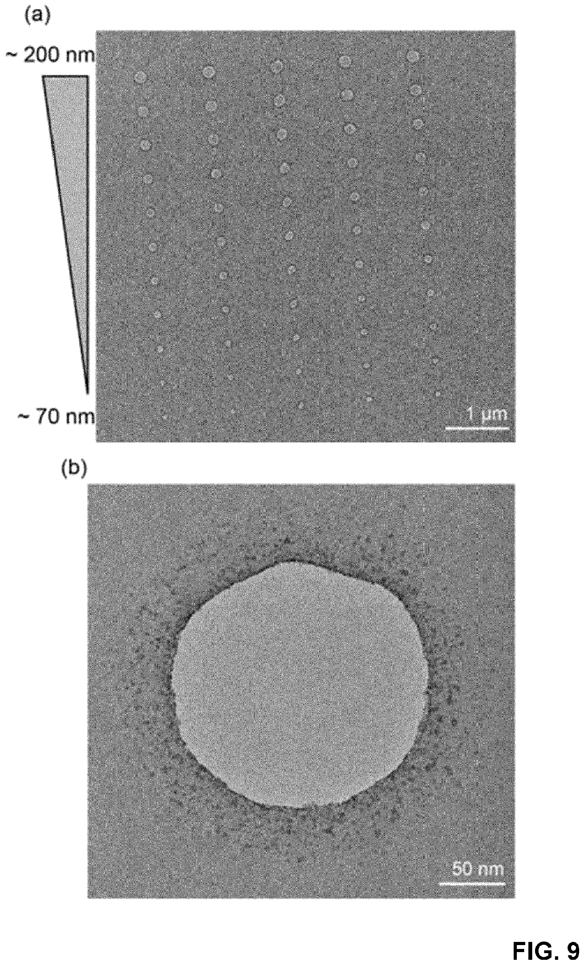

Fig. 9 shows elimination of the selective growth effect on silicon nitride template apertures by extending the purging period by ten times compared with the standard process for growing $HfO_2$ stalactite. (a) TEM image of the modified growth process (6 extend-purging cycles) on silicon nitride template aperture with increasing diameters. (b) TEM image of a single silicon nitride aperture. Dark spots indicate that only a limited amount of $HfO_2$ is growing on the aperture;

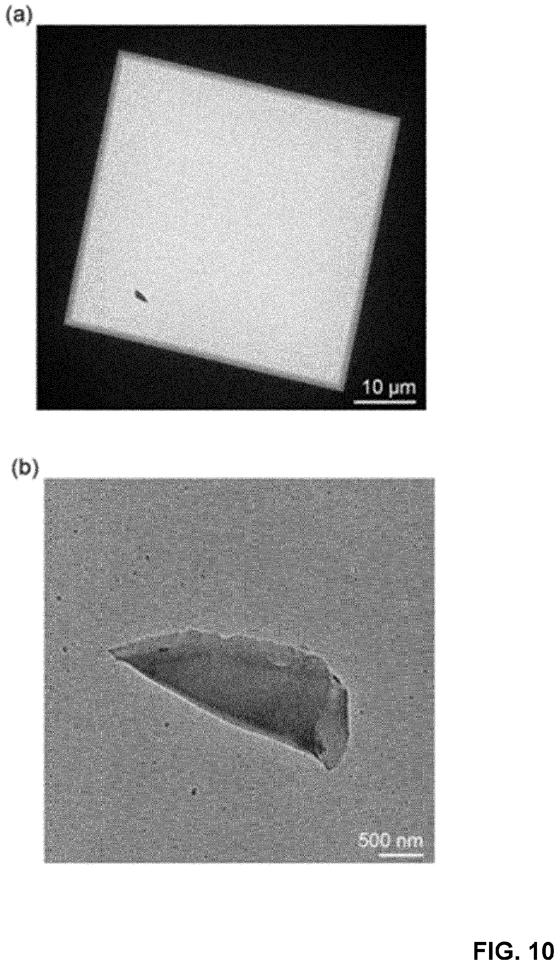

Fig. 10 shows stalactite $HfO_2$ growth process on a silicon nitride freestanding membrane without apertures. (a) No obvious high contrast structure on silicon nitride membrane indicates no obvious selective growth process occurs on the membrane. (b) The magnification of contamination on the silicon nitride membrane;

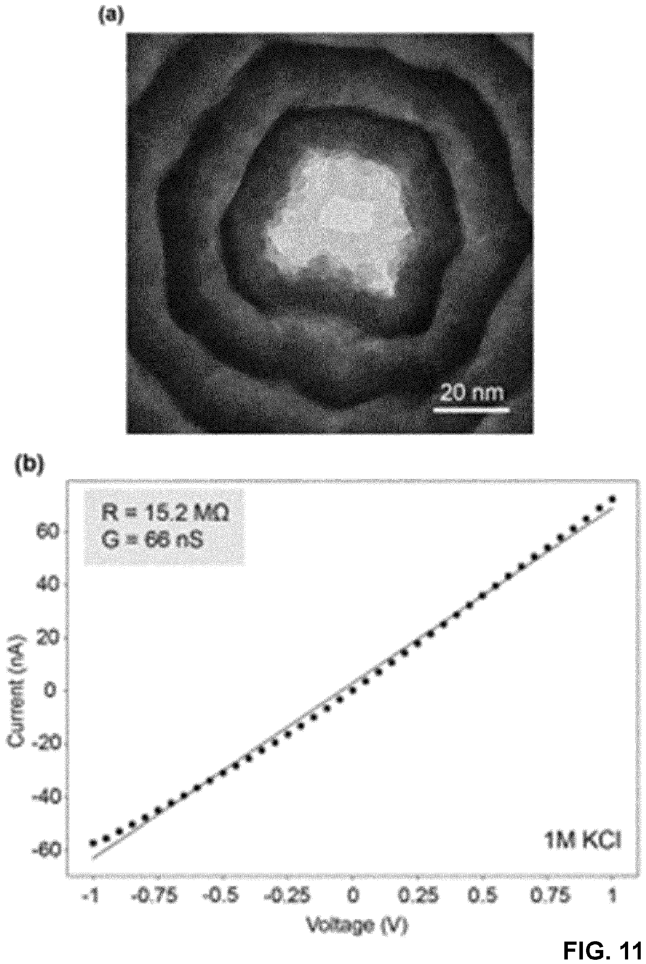

Fig. 11 shows (a) TEM image of stalactite nanopore sensing device for the DNA translocation experiment, (b) I-V measurement of the same device (±1V) in 1M KCl and an open pore conductance (G) is extracted from the linear fit to the I-V response;

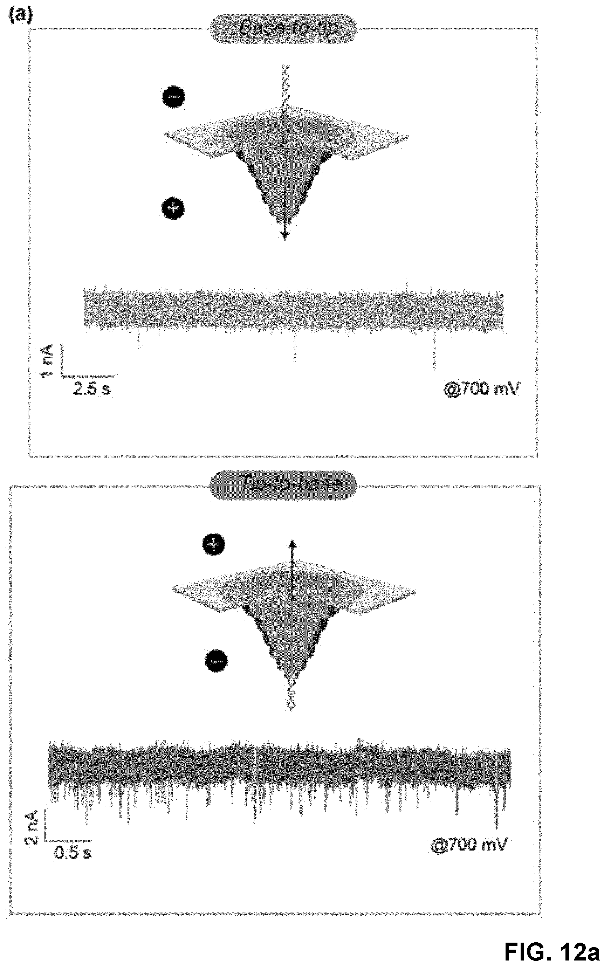

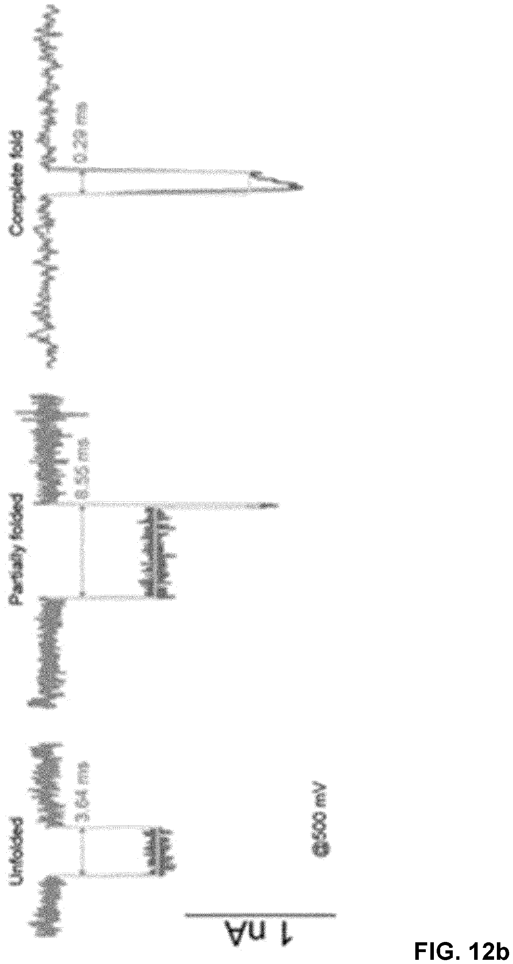

Fig. 12 shows (a) schematic showing comparison of double-stranded DNA translocation via tip-to-base and base-to-tip configuration on a stalactite-like nanopore device. The open pore current trace (below) shows a 2 kbp DNA translocation using stalactite-like HfO2 nanopore (~16 nm) at 700 mV. (b) Examples of 2 kbp individual DNA translocation events showing unfolded, partially folded, and completely folded conformation of dsDNA molecule translocating from stalactite-like nanopore in tip-to-base configuration at 500 mV. The light gray line represents CUSUM-fit to the individual translocation event;

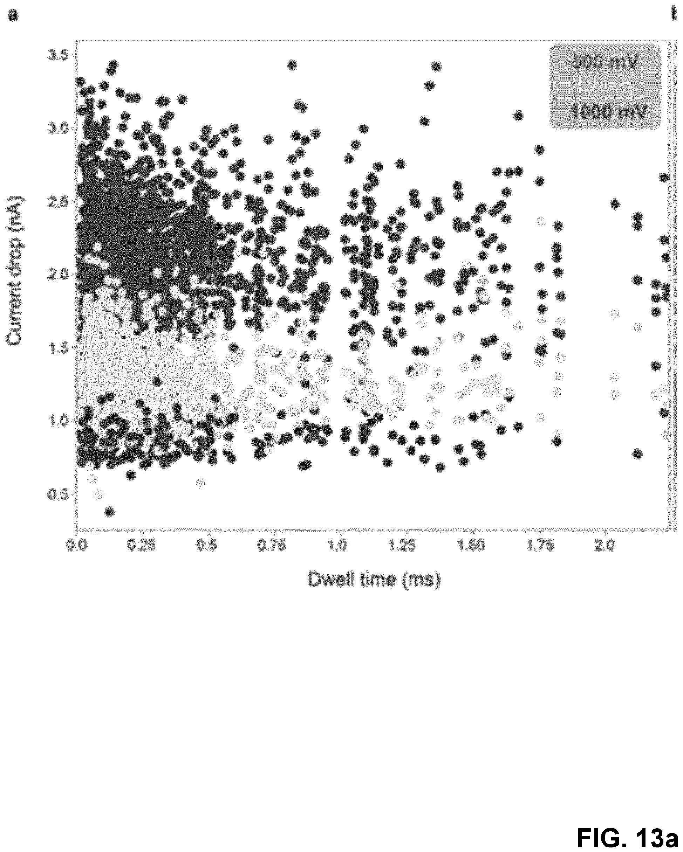

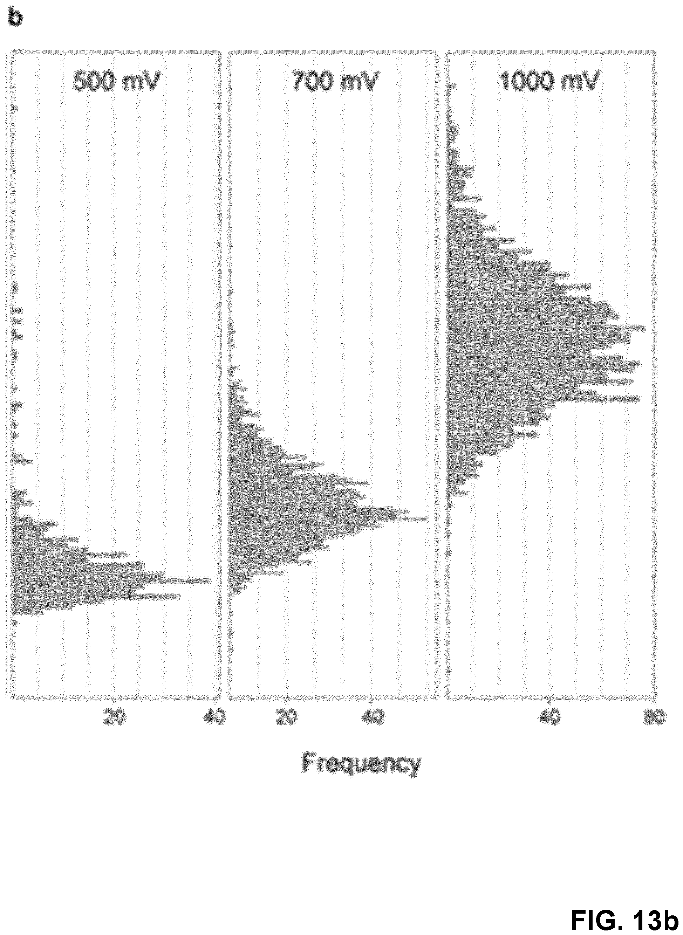

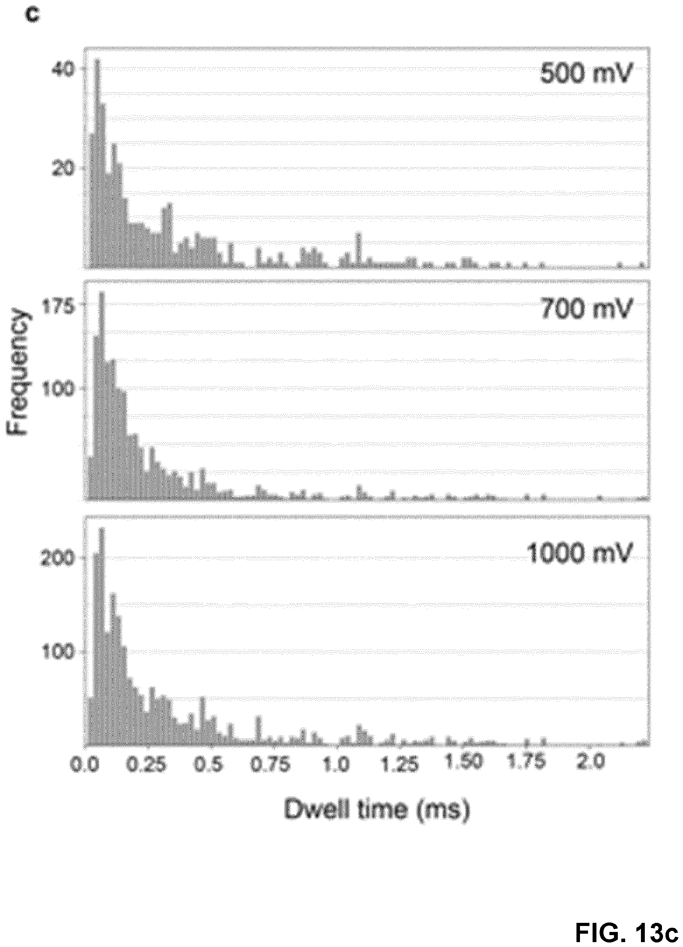

Fig. 13    shows statistics of 2 kbp DNA translocation via tip-to-base configuration. Each event was fitted using CUSUM-algorithm[36,37] and defined as events at different voltages. (a) Scatter plot of individual DNA translocation events at different voltages (500, 700, and 1000 mV). The number of fitted events recorded is 426, 1626, and 2327 events at 500, 700, and 1000 mV respectively. (b) The mean current drop increases while as seen in (c) the dwell time decreases as a function of increasing voltage;

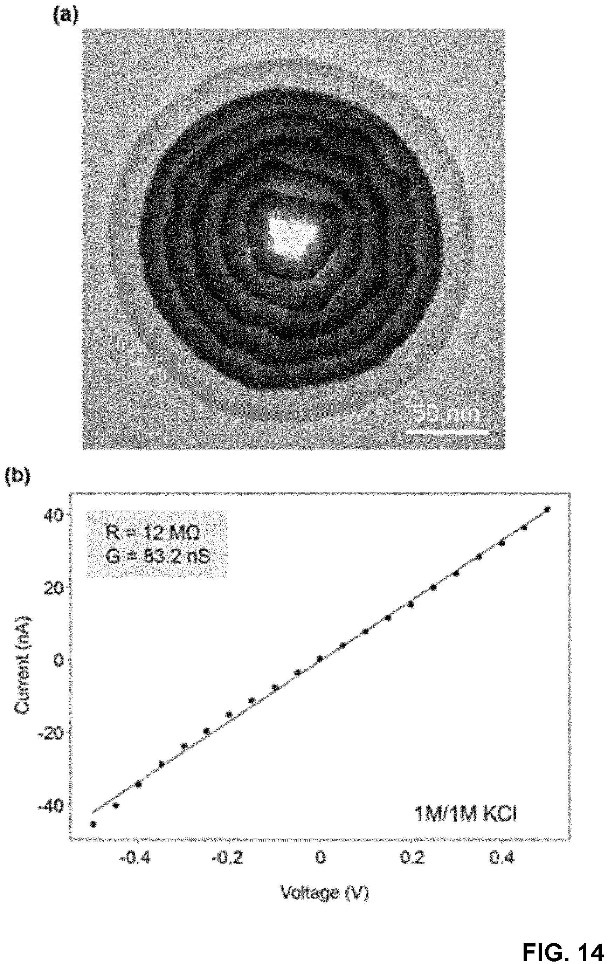

Fig. 14    shows (a) TEM image of $HfO_2$ stalactite nanopore device in the form of an osmotic power generator used for the osmotic power generation. (b) I-V measurement of the same device ($\pm 0.5$V) in symmetric 1M/1M KCl and an open pore conductance (G) is extracted from the linear fit to the I-V response;

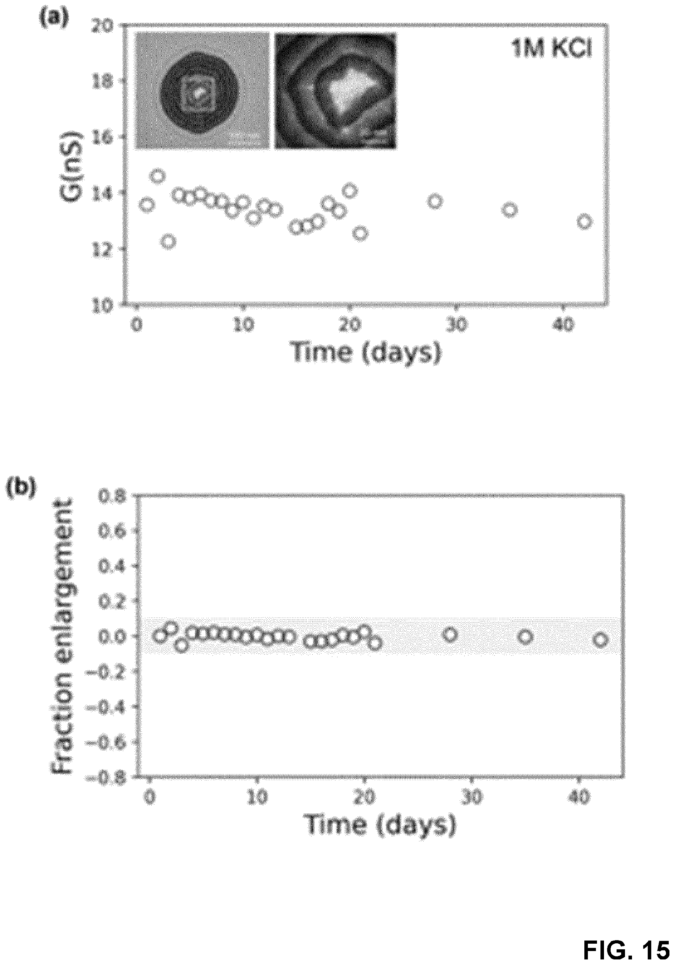

Fig. 15    shows stability analysis of stalactite-like nanopore device in aqueous 1M KCl salt solution. (a) Open-pore conductance for a total period of 42 days (6 weeks) in an aqueous 1M KCl salt solution. Inset shows a TEM image of the stalactite nanopore device. The device was always stored in 1M KCl and sealed between measurements. Each time the device was flushed with a fresh 1M KCl solution for measurements. We performed multiple I-V measurements ($\pm 500$ mV) in order to analyze the wetting state and calculate the open pore conductance of the pore. (b) Fraction enlargement (gray-inset with <10% change) of the stalactite-like nanopore after incubation in high salt (1M KCl) concentration shows high pore stability for >40 days.

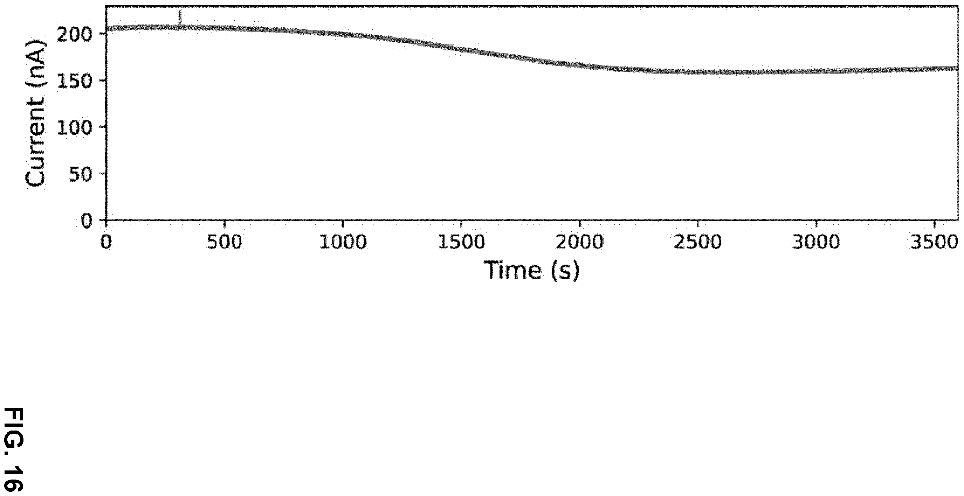

Fig. 16    shows stability test of simulated RED setup. Stalactites $HfO_2$ nanopore arrays are measured under 1000 mM / 10 mM KCl solution at around pH 5 with the same orientation as Fig. 5b.

## DESCRIPTION OF PREFERRED EMBODIMENTS

Nature provides a wide range of self-assembled structures from the nanoscale to the macroscale. Under the right thermodynamic conditions and with the appropriate material supply, structures like stalactites, icicles, and corals can grow. However, the natural growth process is time-consuming.

With reference to the figures the fast, nature-inspired method according to the invention for growing structures such as stalactite nanopores using heterogeneous atomic deposition of hafnium dioxide at the orifice of templated silicon nitride apertures is illustrated. These stalactite-like structures combine the benefits of reduced sensing region typically for 2D material nanopores with the asymmetric geometry of capillaries, resulting in ionic selectivity, stability, and scalability. The proposed growing method provides an adaptable nanopore platform for basic and applied nanofluidic research, including biosensing, energy science, and filtration technologies.

In particular, we introduce asymmetric conical nanopores, termed stalactite-pores, with unique structures compared to existing solid-state nanopores. The fabrication method of stalactite-nanopores is directly inspired by nature. Myriads of examples exist when periodic thermodynamic conditions permit peculiar structures such as corals, stalactites, brinicles, and icicles[18]. These naturally assembled objects' structures are formed due to cyclical supplies of precursors and changes in the thermodynamic parameters at the point of growth. This strategy inspired us to use a similar approach applied to nanopores. Unlike natural objects, we used state-of-the-art cleanroom techniques to facilitate the process and dramatically improve its pace. Using a templated silicon nitride aperture, herein also referred to as a silicon nitride template aperture, as a starting point of the growth, we demonstrate the anisotropic growth of periodic hafnium-oxide rings, resulting in shallow conical ladder-shaped stalactite nanostructures (Fig. 1). This fabrication method allows one to create highly-asymmetric nanopores with on-demand pore sizes from single-digit to hundreds of nanometers, providing a new platform for nanofluidic research with good potential for scalability.

## Experimental

Typical stalactites and stalagmites are structures determined by the precipitation of calcium carbonate from the liquid flowing or dropping on their surfaces (Fig. 1a). The combination of material precipitation, fluid dynamics, evaporation, and periodic thermodynamic conditions determines the growth and shape of natural stalactites and stalagmites[19]. To implement the natural process in artificial nanopore growth, we used templated silicon-nitride (SiN) apertures (Figures 7 and 8), which were pre-patterned with electronic-beam lithography and dry etching to form periodic templated apertures (Fig. 1b, inset). We used various templates with a single pore or a multipore array of the same size or gradient aperture size. To realize the stalactite structures at the nanoscale (Fig. 1b), the templated SiN apertures were placed into a reaction chamber, typically used for atomic-layer deposition (ALD) (Fig. 1c), yet the growth regimes are different.

Adapting the standard ALD process, the precursors, in the present examples water vapor and tetrakis(ethylmethylamido)hafnium, were used for $HfO_2$

growth, varying the pulse duration. Templated silicon nitride aperture on a single chip substrate served as a nanoreactor for the material precipitation (Fig. 1c). Due to the heterogeneity of the substrate caused by the presence of aperture(s) and presumably due to the local concentration gradients of precursors, precipitation was mainly at the orifice of the aperture(s). The growth rate at the aperture orifice was two orders of magnitude higher than the aperture-free region. As a result, $HfO_2$ stalactite nanopores were forming at each aperture (Fig. 1d), presumably because the precursor molecules have not had enough time to diffuse away from being trapped by the compartment formed by the aperture. Then the time between the pulses increases, the molecules have sufficient time to diffuse, and the standard ALD growth mode takes place (Figures 9 and 10).

## Results

The typical stalactite $HfO_2$ nanopore is shown in the transmission electron microscopy (TEM) micrograph in Figure 1b. The pore array is shown on top, and the single pore is enlarged on the bottom. The gray-and-black contrast demonstrates the periodic structure of the pore. Each ring corresponds to one growth cycle. Each pore has a 3D structure. The 3D sketch of a single pore is shown in Figure 1d. The pore has a conical shape of the length L, defined by the number of rings. The pore base diameter a is defined by the initial aperture diameter. The pore tip diameter d depends on the number of cycles and can be as low as 3 nm (Fig. 1b, bottom).

TEM images, including energy-dispersive X-ray (EDX) mapping, are shown in Fig. 2. The high-resolution TEM micrograph (Fig. 2a(I)) shows that a single $HfO_2$ stalactite has a periodic step-like structure similar to that observed in natural stalactites (Fig. 1a). The pore shown on the picture has eight rings. The rings grow in the direction from the pore perimeter to the center so that a preceding ring is a template for the next ring with a gradually reducing diameter. The energy-dispersive X-ray spectroscopy analysis of the side-view projection prepared with a focused ion beam shows the elemental composition of the stalactite (Fig. 2a(II)). The signal from hafnium atoms corresponds to the stalactite body, whereas nitrogen atoms correspond to the silicon nitride membrane. High-angle annular dark-field imaging (HAADF) is shown in Figure 2b(I). The EDX mapping shows heterogeneity in absolute values (Fig. 2b(II)), which disappears in the thickness-normalized signal (Fig. 2b(III)). This fact indicates the even distribution of hafnium within the stalactite.

There are two ways of tuning the pore diameter. The first is choosing the number of growth cycles (Fig. 2c). For example, one, three, and five cycles result in zero, two, and four rings in the $HfO_2$ stalactite nanostructure, respectively. Therefore, each growth cycle except the first one adds a ring to the stalactite until it clogs. In other words, n cycles of precursor supply result in n-1 rings. The second way to control the stalactite dimensions is by varying the diameter of the templated silicon-nitride apertures (Fig. 2d). The decrease of the aperture diameter results in smaller nanopores for the same number of growth cycles. The fine-tuning of the growth cycles and choosing the apertures' diameter allows one to obtain the stalactite nanopore with desired dimensions (Fig. 1d). However, one has to carefully adjust the growth parameters to prevent the overgrowth of the stalactite nanostructure and the consequent termination of the nanopore due to the overfilling of its inner volume.

## Stalactite nanopore in nanofluidic experiments

A distinctive feature of the stalactite nanopore is its asymmetry. The latter was demonstrated to influence the charge and mass transport through the pore with and without an electric field, with and without a concentration gradient[20-22]. Particularly, the tapering geometry creates nonuniformity of the electric field distribution, helping separate charges and, for example, produce electricity from the concentration gradients[16] or affecting the translocation rate of the biomolecules through the pore[23,24]. To understand the impact of the asymmetric geometry on the ionic and molecular transport through the stalactite nanopore and estimate its 'efficiency', we performed DNA translocation and reverse electrodialysis experiments. The latter was made on both a single pore and a nanopore array.

## Biosensing: DNA translocations

Figure 3a shows a layout for the DNA translocation experiment. The insets demonstrate an electric current through the pore during a single DNA translocation event for two cases: tip-to-base (left) and base-to-tip (right) directions of translocation. The object of interest is the difference in the translocation frequency between two directions with charged DNA driven by an electric field. We used two voltage levels of 0.7 and 1 V. The current-voltage characteristics are given in Figure 11, and details of translocation comparison are shown in Figures 12 and 13. We found that the asymmetric shape of the pore yields asymmetry in the DNA translocation (Fig. 3b). The concentration-normalized DNA translocation rate for the *tip-to-base* direction is almost an order of magnitude higher than for the *base-to-tip* direction. This difference is presumably due to the higher energy barrier for the latter case caused by the nonuniform electric field distribution[25] and the electroosmotic flow[26] that the negatively-charged DNA molecule overcomes during the translocation. Our COMSOL™ simulation showed that the pore has an electric field gradient along the symmetry axis (Fig. 3c). This gradient exists even without a concentration gradient. Yet, the latter increases the asymmetry. The nonuniform electric field of the pore increases the chances for the negatively charged DNA molecule to be trapped from the tip side. As the effect has a pure electrostatic nature, we expect the pore asymmetry will affect the translocation of any charged molecules passing through the pore. This effect can be used for biomolecule characterization and separation.

Reverse electrodialysis

Asymmetric pores empower energy harvesting. Figure 4a shows the schematics of the reverse electrodialysis experiment, which has been designed to demonstrate the osmotic energy conversion[27]. The setup consisted of a single stalactite nanopore in the membrane separating two volumes with aqueous KCl solutions. The concentration gradient between the opposite sides of the membrane was changing within three orders of magnitude. Two Ag/AgCl electrodes were recording the ionic current. The efficiency of ions rectification was compared for two cases of the tip-to-base and the base-to-tip flow directions (Fig. 4b). The experiments were repeated several times using stalactite nanopores with a tip diameter of $15\pm5$ nm (Figure 14). The measured I-V characteristics are shown in Figure 4c (see Methods for details). The osmotic current $I_o$ and the osmotic potential $V_o$ were obtained using a standard procedure[28].

The tip-to-base flow direction showed higher $V_o$ and $I_o$ than the base-to-tip flow (Fig. 4d). This effect originates from the difference in the spatial charge distribution (Fig. 4e) for the two cases. Different ion rectification of the stalactite nanopore occurs due to the differential diffusion of cations and anions across the membrane in the presence of the concentration gradient. The difference between the values of osmotic current (left) and osmotic potential (right) for the tip-to-base and base-to-tip directions of the concentration gradients comes from the asymmetric geometry. The lower concentration at the base side leads to a broader electrical double layer (EDL) at the inner pore wall (Fig. 4e, left), which results in higher ion selectivity by the pore. The lower concentration on the tip side also yields a widening of EDL, but on the flat substrate. As a result, the electric field distribution inside the pore is not perturbed, and the selectivity drops. Thus, geometric asymmetry provides additional benefits for the osmotic energy conversion compared to the cylindrical nanopores. The surface charges at the pore walls can vary by surface modification. This treatment can additionally increase the efficiency of osmotic power generation.

Figure 4f shows the dependence of $I_o$ on concentration gradients. Three curves are measured at different pH and showed high sensitivity of the osmotic current on the surface charge, which is known to be pH dependent. Although the highest osmotic current is observed for pH = 9, the $HfO_2$ nanopore shows relatively good osmotic power at neutral pH. This fact positively distinguishes our assembly as it can be used for natural applications, such as, e.g. energy harvesting at the see-river contact in which water has neutral pH. Our experiment showed that a single conical $HfO_2$ nanopore gen-

erates 0.4 nW of power at pH = 5 and 1 nW at pH = 9. These values are higher compared to the previously reported for nanofluidic systems[28,29]. Moreover, stalactite nanopores are very stable due to the inert chemical nature of $HfO_2$. Our nanopores showed remarkable stability in 1 M KCl solution for 42 days. The variation of pore conductance was within 10% (Figure 15). Modifying the surface of our $HfO_2$ nanopore by deposition of superhydrophobic materials can also increase osmotic power generation.

Osmotic energy conversion with nanopore arrays

Practical applications, such as osmotic power generation, energy storage, filtration, and separation, require high throughput and, thus, arrays of nanopores rather than a single pore[15,30,31]. To demonstrate the scaling potential of stalactite nanopores, we used templated silicon nitride aperture arrays with 900 nanopores (Fig. 5a, bottom) at which $HfO_2$ was grown by the same procedure as for a single pore. Arrays consisted of the equivalent single pores of $15\pm5$ nm (Fig. 5a, top). We found that the transition from a single pore to an array does not affect the precision of the fabrication and quality of the nanopores.

Figure 5b shows the experimental setup layout. It consists of the osmotic battery with the nanopore membrane, the ammeter, and the imitation of the working load. We used a tip-to-base concentration gradient direction that showed a higher osmotic current on a single pore (Fig. 4). Note the device showed a high output current stability for more than one hour under a 1-to-0.1 M KCl concentration gradient (Figure 16). Varying the load resistance, we measured the device power density (Fig. 5c) and the current density (Fig. 5d) for three different salinity gradients. The device showed the highest power density at $C_{max}/C_{min} = 10^3$ and a load resistance of 1 MOhm. Taking into account the membrane surface of 400 $\mu m^2$, we get 20 $W/m^2$ from this demonstration device. Note that the $HfO_2$ pores permit the deposition of any materials on their surface by an additional standard atomic-layer deposition technique, which is a cheap method for surface modification. Further studies in this direction can improve the functional properties of our stalactite nanopores' surface charge.

Conclusion

We demonstrated a nature-inspired controllable fabrication of the stalactite nanopores made by the self-assembled anisotropic growth of $HfO_2$ shallow nanostructure on templated silicon nitride apertures. By tuning the diameter of templated silicon nitride apertures and the number of cycles of precursor supply, we demonstrated nanopore devices with a diameter from single-digit to several hundreds of nanometers. TEM imaging and EDX mapping confirmed the selective growth of $HfO_2$ stalactite-like structures inside silicon nitride apertures, which differs from the standard ALD. We showed that nanoconfinement enhances the local growth rate by two orders of magnitude compared to the conventional ALD process. The advantage of asymmetric stalactite nanopores has been demonstrated through DNA translocation and reversed electrodialysis. Both experiments demonstrated better DNA capture and osmotic energy conversion efficiency for the tip-to-base direction of the concentration gradient. Using the array of 900 $\mu m^2$ with 900 nanopores, we demonstrated an efficient osmotic power generation. Due to the extremely inert nature of $HfO_2$, the device degradation rate was found to be less than 10% per month, which provides state-of-the-art stability suitable for real-life applications. The pores can be further modified by standard ALD material deposition to obtain the optimal diameter and surface charge for a target application. The proposed method provides a new adaptable platform in terms of geometry and material composition for applied and basic nanofluidic research, including bioengineering, iontronics, energy science, and filtration technologies.

## Methods

### Samples preparation

We used standard 4-inch boron-doped 380 ± 10 $\mu$m-thick silicon (1-10 Ohm·cm) template wafers supplied by the EPFL Center of MicroNanoTechnology (CMi). The layers of 60 nm of dry silicon oxide and 20 nm of low-stress silicon nitride were formed at the polished surfaces using Centrotherm furnaces at the CMi clean-room facility. The nanopores in the template were patterned by using e-beam lithography with consequent dry etching[32]. Each wafer had either a single pore or an array of nanopores with the apertures varying from 100 to 200 nm. To grow the hafnium-oxide 'stalactite' at the orifice of the silicon nitride aperture, we used a commercial BeneQ TFS200 atomic layer deposition (ALD) setup. To initiate the $HfO_2$ precipitation, we used two precursors: pure water vapor and 99.99% Tetrakis-(ethylmethylamido)-hafnium (IV), purchased from Sigma Aldrich. The chamber temperature was set to 200°C, and the precursors' temperature was set to 80°C following the recipe described before[33]. The precursors' injection was repeated several times to get the required number of rings of the 'stalactite.' The chamber was purged with nitrogen for 2 seconds between the cycles. The growth rate for this recipe and a standard flat surface was 1.01 ± 0.05 Å/cycle. To heat the SiN template, we used the Inox-316L substrate. Each sample was pre-heated for 15 minutes before the growth cycle. For the biomolecules translocation experiment, we used 2-kbp double-stranded DNA in 1 M aqueous solution of KCl.

### Sample characterization

The samples were imaged using Osiris™, Talos™, and Titan™ transmission electron microscopy (TEM) instruments by FEI Company at 200 kV. We used three different built-in working modes: the High-Resolution TEM (HRTEM), the Scanning TEM (STEM) with Energy-dispersive X-ray spectroscopy (EDX), and the High-Resolution STEM with Energy-dispersive X-ray spectroscopy. EDX mappings of the single nanopore (Fig. 2e) were obtained using the data from Figure 2d by re-normalizing the signal to the thickness of the sample, measured by atomic force microscopy (AFM). To observe the 'side view' of the icicle nanopore (Fig. 2b), we used Focused Ion Beam (FIB) cutting. The SiN membrane with the icicle pores was cut perpendicularly to the wafer surface by Ga ions using the Zeiss Crossbeam FIB system. The cross-section was imaged using Scanning Electron Microscopy (SEM) technique. SEM images of the icicle-pore arrays (Fig. 1b) were obtained by the same machine at 1.5 kV.

### Numerical simulations

To visualize the ions distribution and the electric field distribution within the icicle nanopores, we used the COMSOL Multiphysics® package. The electric field distribution was simulated using the finite element method (FEM). The calculations were made in a stationary regime with similar dimensions from STEM analysis. The electric field distribution was calculated in different concentration conditions used in the real DNA translocation and reverseelectrodialysis (RED) experiments detailed in Supporting Information Note 1.

## Supporting Information Note 1

### 1. Finite element simulations

We solve the Poisson-Nernst-Planck equation for the electric potential and the K+/Cl- ion concentration using the finite-element method (COMSOL Multiphysics 6.0). The corresponding equations coupling the electric potential to the ion concentrations are based on the Poisson and Nernst-Planck equations. In all simulations, we considered a pore of a similar shape of STEM analysis using a 2D axisymmetric geometry presented in Figure 6. Axisymmetric geometry of the nanopore with staired shape (gray zone) used for the numerical resolution of the corresponding Poisson and Nernst-Planck equations using a finite-element solver (COMSOL). Each stair is 20 nm wide and 20 nm deep with a 2 nm fillet on all corners to achieve better mesh distribution. The dashed line represents the ideal infinite boundary of the structure, and the solid line represents the nanopore boundaries that were applied with surface charge during the simulation[34,35].

Based on these models, two kinds of simulation have been achieved with the same geometry and different conditions. The surface charge is set at -0.05 $C/m^2$, and the relative permittivity is set at 80.1. In the DNA translocation section, we calculate the absolute value of the local electric field intensity based on the addition of its gradience in r and z directions. The electric field is described by the vector sum of the partial differentials of the electric potential in the r and z directions. The local electric field intensity of two conditions, which represents the positive or negative voltage applied on this system, are shown with a logarithmic scale with a shared axis range.

$$|E| = \left| \frac{\partial V}{\partial r} + \frac{\partial V}{\partial z} \right|$$

In the reversed electrodialysis part, the concentrations at the two reservoirs are different to demonstrate a simulated spontaneous diffusion process. We define a localized relative distribution bias to represent the local concentration difference between cation and anion. The value of it can be taken as an index to show which region of nanopore has net charge flux that will contribute to net ionic current introduced by diffusion:

$$Local\ distribution\ bias = \left| \frac{c_{K^+} - c_{Cl^-}}{c_{K^+} + c_{Cl^-}} \right|$$

References

1. Kasianowicz, J. J., Brandin, E., Branton, D. & Deamer, D. W. Characterization of individual polynucleotide molecules using a membrane channel. Proc. Natl. Acad. Sci. 93, 13770-13773 (1996).

2. Stein, D., Kruithof, M. & Dekker, C. Surface-Charge-Governed Ion Transport in Nanofluidic Channels. Phys. Rev. Lett. 93, 035901 (2004).

3. Dekker, C. Solid-state nanopores. Nat. Nanotechnol. 2, 209-215 (2007).

4. Robin, P. et al. Long-term memory and synapse-like dynamics in two-dimensional nanofluidic channels. Science 379, 161-167 (2023).

5. Teng, Y. et al. Bioinspired nervous signal transmission system based on two-dimensional laminar nanofluidics: From electronics to ionics. Proc. Natl. Acad. Sci. 117, 16743-16748 (2020).

6. Xue, L. et al. Solid-state nanopore sensors. Nat. Rev. Mater. 5, 931-951 (2020).

7. Acar, E. T., Buchsbaum, S. F., Combs, C., Fornasiero, F. & Siwy, Z. S. Biomimetic potassium-selective nanopores. Sci. Adv. 5, eaav2568 (2019).

8. Fang, A., Kroenlein, K., Riccardi, D. & Smolyanitsky, A. Highly mechanosensitive ion channels from graphene-embedded crown ethers. Nat. Mater. 18, 76-81 (2019).

9. Bocquet, L. Nanofluidics coming of age. Nat. Mater. 19, 254-256 (2020).

10. Cressiot, B., Greive, S. J., Mojtabavi, M., Antson, A. A. & Wanunu, M. Thermostable virus portal proteins as reprogrammable adapters for solid-state nanopore sensors. Nat. Commun. 9, 4652 (2018).

11. Hall, A. R. et al. Hybrid pore formation by directed insertion of α-haemolysin into solid-state nanopores. Nat. Nanotechnol. 5, 874-877 (2010).

12. Chou, Y.-C., Masih Das, P., Monos, D. S. & Drndic, M. Lifetime and Stability of Silicon Nitride Nanopores and Nanopore Arrays for Ionic Measurements. ACS Nano 14, 6715-6728 (2020).

13. He, Y., Tsutsui, M., Zhou, Y. & Miao, X.-S. Solid-state nanopore systems: from materials to applications. NPG Asia Mater. 13, 1-26 (2021).

14. Liu, H., Zhou, Q., Wang, W., Fang, F. & Zhang, J. Solid-State Nanopore Array: Manufacturing and Applications. Small 19, 2205680 (2023).

15. Gadaleta, A. et al. Sub-additive ionic transport across arrays of solid-state nanopores. Phys. Fluids 26, 012005 (2014).

16. Ma, L. et al. Modulation of Ionic Current Rectification in Ultrashort Conical Nanopores. Anal. Chem. 92, 16188-16196 (2020).

17. Macha, M., Marion, S., Nandigana, V. V. R. & Radenovic, A. 2D materials as an emerging platform for nanopore-based power generation. Nat. Rev. Mater. 4, 588-605 (2019).

18. Katiyar, N. K., Goel, G., Hawi, S. & Goel, S. Nature-inspired materials: Emerging trends and prospects. NPG Asia Mater. 13, 1-16 (2021).

19. Black, D. M. Cave Pearls in Carlsbad Caverns. Sci. Mon. 74, 206-210 (1952).

20. Shaw, R. S., Packard, N., Schröter, M. & Swinney, H. L. Geometry-induced asymmetric diffusion. Proc. Natl. Acad. Sci. 104, 9580-9584 (2007).

21. Zhang, Z. et al. Ultrathin and Ion-Selective Janus Membranes for High-Performance Osmotic Energy Conversion. J. Am. Chem. Soc. 139, 8905-8914 (2017).

22. Hsu, J.-P. et al. Unraveling the Anomalous Surface-Charge-Dependent Osmotic Power Using a Single Funnel-Shaped Nanochannel. ACS Nano 13, 13374-13381 (2019).

23. Liu, X., Skanata, M. M. & Stein, D. Entropic cages for trapping DNA near a nanopore. Nat. Commun. 6, 6222 (2015).

24. Freedman, K. J. et al. Nanopore sensing at ultralow concentrations using single-molecule dielectrophoretic trapping. Nat. Commun. 7, 10217 (2016).

25. Bell, N. A. W., Muthukumar, M. & Keyser, U. F. Translocation frequency of double-stranded DNA through a solid-state nanopore. Phys. Rev. E 93, 022401 (2016).

26. Luo, L., Holden, D. A. & White, H. S. Negative Differential Electrolyte Resistance in a Solid-State Nanopore Resulting from Electroosmotic Flow Bistability. ACS Nano 8, 3023-3030 (2014).

27. Zhang, Z., Wen, L. & Jiang, L. Nanofluidics for osmotic energy conversion. Nat. Rev. Mater. 6, 622-639 (2021).

28. Feng, J. et al. Single-layer MoS2 nanopores as nanopower generators. Nature 536, 197-200 (2016).

29. Siria, A. et al. Giant osmotic energy conversion measured in a single transmembrane boron nitride nanotube. Nature 494, 455-458 (2013).

30. Yazda, K. et al. High Osmotic Power Generation via Nanopore Arrays in Hybrid Hexagonal Boron Nitride/Silicon Nitride Membranes. Nano Lett. 21, 4152-4159 (2021).

31. Tsutsui, M., Yokota, K., Leong, I. W., He, Y. & Kawai, T. Sparse multi-nanopore osmotic power generators. Cell Rep. Phys. Sci. 3, 101065 (2022).

32. Thakur, M. et al. Wafer-Scale Fabrication of Nanopore Devices for Single-Molecule DNA Biosensing using MoS2. Small Methods 4, 2000072 (2020).

33. Hausmann, D. M., Kim, E., Becker, J. & Gordon, R. G. Atomic Layer Deposition of Hafnium and Zirconium Oxides Using Metal Amide Precursors. Chem. Mater. 14, 4350-4358 (2002).

34. Traversi, F. *et al.* Detecting the translocation of DNA through a nanopore using graphene nanoribbons. *Nat. Nanotechnol.* **8**, 939-945 (2013).

35. Davis, S. J. *et al.* Pressure-Induced Enlargement and Ionic Current Rectification in Symmetric Nanopores. *Nano Lett.* **20**, 8089-8095 (2020).

36. Raillon, C., Granjon, P., Graf, M., Steinbock, L. J. & Radenovic, A. Fast and automatic processing of multi-level events in nanopore translocation experiments. *Nanoscale* **4**, 4916-4924 (2012).

37. Graf, M. *et al.* Fabrication and practical applications of molybdenum disulfide nanopores. *Nat. Protoc.* **14**, 1130-1168 (2019).

**Claims**

1. A method of forming a structure, preferably a pore, comprising the steps of:

   - Providing a template comprising at least one template aperture, and
   - Growing at least one material at an orifice of the template aperture by depositing the material, whereby the structure is formed.

2. The method according to claim 1, wherein the material is grown by supplying at least two precursors in two or more supply cycles, and

   wherein, in a first supply cycle, the precursors react with the orifice of the template aperture, whereby the material is deposited at the orifice of the template aperture, and
   wherein, in subsequent supply cycles, the precursors react with the material being deposited in a previous supply cycle, whereby the deposited material grows in growth cycles and preferably in the form of rings.

3. The method according to claim 2, wherein the length of the structure is determined by the number of supply cycles, and/or
   wherein the structure extends from the orifice of the template aperture along a structure axis and terminates in a structure tip, the size of the structure tip being determined by the number of supply cycles.

4. The method according to any one of the preceding claims, wherein the structure is of an asymmetric shape and/or a conical shape.

5. The method according to any one of the preceding claims, wherein the template is a suspended membrane arranged on a substrate comprising at least one opening,

   wherein the suspended membrane spans the opening of the substrate,
   wherein the material to be deposited is supplied to the suspended membrane and entering the opening of the substrate via the suspended membrane, and
   wherein the material is growing inside the opening of the substrate.

6. The method according to any one of the preceding claims, wherein the material is furthermore deposited on the template in aperture-free regions of the template, and
   wherein the deposited material preferably forms a layer on the template.

7. The method according to any one of the preceding claims, wherein the template aperture is pre-patterned by lithography, in particular by electron-beam lithography, photolithography, deep UV lithography or extreme UV lithography, and/or
   wherein the template aperture is formed by etching, in particular dry etching.

8. The method according to any one of the preceding claims, wherein the template comprises a single template aperture, or
   wherein the template comprises a plurality of template apertures.

9. The method according to claim 8, wherein the plurality of template apertures are arranged evenly spaced or unevenly spaced on the template, and/or
   wherein sizes of the orifices of the plurality of template apertures are the same or different from one another.

10. The method according to any one of the preceding claims, wherein a surface property of the structure is modified, and

wherein the surface property of the structure is preferably modified by depositing at least one surface modifier on the surface of the structure.

11. An assembly comprising or consisting of a template and at least one structure, in particular at least one pore, formed in the method according to any one of the preceding claims.

12. An osmotic power generator comprising:

- at least one active membrane;
- at least a first compartment being disposed on the first side of the active membrane and being configured to receive a first electrolyte; and
- at least a second compartment being disposed on a second side of the active membrane and being configured to receive a second electrolyte,

wherein the first electrolyte has greater ionic strength than the second electrolyte, **characterized in that** the active membrane comprises or consists of the assembly as claimed in claim 11, wherein the structure is a pore, and wherein the pore is configured to allow ions of the first electrolyte to pass from the first compartment to the second compartment by diffusion, whereby osmotic power can be generated.

13. A method of generating osmotic power comprising the steps of:

- Providing at least one osmotic power generator as claimed in claim 12;
- Supplying the first electrolyte into the first compartment; and
- Supplying the second electrolyte into the second compartment,

wherein the first electrolyte has greater ionic strength than the second electrolyte, and wherein ions of the first electrolyte pass through the pore from the first compartment to the second compartment by diffusion, whereby osmotic power is generated.

14. A sensing device for sensing an analyte comprising:

- at least one active membrane;
- at least a first compartment being disposed on the first side of the active membrane and being configured to receive a first electrolyte comprising the analyte;
- at least a second compartment being disposed on a second side of the active membrane and being configured to receive a second electrolyte; and

- at least a first electrode being in electrical connection with the first compartment and a second electrode being in electrical connection with the second compartment, **characterized in that** the active membrane comprises or consists of the assembly as claimed in claim 11, wherein the structure is a pore, and wherein the pore is configured to allow ions of the first and second electrolyte to pass between the first compartment and the second compartment upon the application of a potential difference between the first electrode and the second electrode, whereby an ionic current is changeable by a translocation of the analyte through the pore.

15. A method of sensing an analyte comprising the steps of:

- Providing at least one sensing device as claimed in claim 14;
- Supplying the first electrolyte comprising or consisting of the analyte into the first compartment;
- Supplying the second electrolyte into the second compartment; and
- Applying a potential difference between the first electrode and the second electrode, wherein ions of the first and second electrolyte pass through the pore between the first compartment and the second compartment, whereby an ionic current is changed by a translocation of the analyte through the pore.

(a)

(b)

500 nm

20 nm

FIG. 1

**(c)**

**(d)**

HfO₂ Stalactites Structure

FIG. 1

(a)

(b)

FIG. 2

**FIG. 2**

FIG. 3

**(b)**

FIG. 3

(c)

1 V

-1 V

Tip-to-base

Base-to-tip

$\log_{10}(V\,m^{-1})$

8.0
7.5
7.0
6.5
6.0
5.5
5.0
4.5
4.0
3.5
3.0

**FIG. 3**

(a)

Electrodes

HfO₂ nanopore device

Electrolyte

(b)

K⁺

Cl⁻

Base-to-tip

Tip-to-base

FIG. 4

**FIG. 4**

**(e)**

**(f)**

**FIG. 4**

**(a)**

FIG. 5

**(b)**

FIG. 5

FIG. 5

FIG. 6

(a)                                              (b)

FIG. 7

(a)

5 µm

(b)

50 nm

**FIG. 8**

(a)

(b)

**FIG. 9**

(a)

(b)

**FIG. 10**

(a)

20 nm

(b)

R = 15.2 MΩ
G = 66 nS

1M KCl

**FIG. 11**

(a)

FIG. 12a

FIG. 12b

a

FIG. 13a

b

FIG. 13b

FIG. 13c

(a)

(b)

FIG. 14

FIG. 15

FIG. 16

Europäisches
Patentamt

European
Patent Office

Office européen
des brevets

**EUROPEAN SEARCH REPORT**

Application Number

EP 23 17 0779

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ULRICH NILS ET AL: "Conical Nanotubes Synthesized by Atomic Layer Deposition of Al2O3, TiO2, and SiO2 in Etched Ion-Track Nanochannels", NANOMATERIALS, vol. 11, no. 8, 21 July 2021 (2021-07-21), page 1874, XP93085500, DOI: 10.3390/nano11081874 | 1-12,14 | INV. B01D69/02 B01D71/02 B01D67/00 F03G7/00 |
| Y | * abstract; figures 1-2 * <br> * page 2, paragraph 3 * <br> ----- | 13,15 | ADD. G01N33/487 H01M8/22 |
| X | US 2010/089866 A1 (PRINZ FRIEDRICH B [US] ET AL) 15 April 2010 (2010-04-15) * paragraph [0037]; claims; figures 2, 4 * <br> ----- | 1,4-11 | |
| X | THANGARAJ VIDHYADEVI ET AL: "Detection of short ssDNA and dsDNA by current-voltage measurements using conical nanopores coated with Al2O3 by atomic layer deposition", MICROCHIMICA ACTA, SPRINGER VIENNA, VIENNA, vol. 183, no. 3, 21 December 2015 (2015-12-21), pages 1011-1017, XP035641228, ISSN: 0026-3672, DOI: 10.1007/S00604-015-1706-2 [retrieved on 2015-12-21] | 1,2, 4-12,14, 15 | |
| Y | * abstract; figure 2 * <br> * page 1013, left-hand column * <br> ----- <br> -/-- | 13,15 | |

**TECHNICAL FIELDS SEARCHED (IPC)**

G01N
H01M
B01D
C23C
F03G

The present search report has been drawn up for all claims

| Place of search | Date of completion of the search | Examiner |
|---|---|---|
| Munich | 27 September 2023 | Veríssimo, Sónia |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

EP 23 17 0779

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LAUCIRICA GREGORIO ET AL: "Shape matters: Enhanced osmotic energy harvesting in bullet-shaped nanochannels", NANO ENERGY, vol. 71, 1 May 2020 (2020-05-01), page 104612, XP93086237, NL ISSN: 2211-2855, DOI: 10.1016/j.nanoen.2020.104612 * Introduction; figure 3 * | 13 | |
| T | CHERNEV ANDREY ET AL: "Nature-Inspired Stalactite Nanopores for Biosensing and Energy Harvesting", ADVANCED MATERIALS, vol. 35, no. 33, 6 July 2023 (2023-07-06), XP93086238, DE ISSN: 0935-9648, DOI: 10.1002/adma.202302827 * the whole document * | | |

|  | TECHNICAL FIELDS SEARCHED (IPC) |

The present search report has been drawn up for all claims

| Place of search | Date of completion of the search | Examiner |
|---|---|---|
| Munich | 27 September 2023 | Veríssimo, Sónia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 0779

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2023

| Patent document cited in search report | Publication date | Patent family member(s) | Publication date |
|---|---|---|---|
| US 2010089866 A1 | 15-04-2010 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KASIANOWICZ, J. J. ; BRANDIN, E. ; BRANTON, D. ; DEAMER, D. W.** Characterization of individual polynucleotide molecules using a membrane channel. *Proc. Natl. Acad. Sci.,* 1996, vol. 93, 13770-13773
- **STEIN, D. ; KRUITHOF, M. ; DEKKER, C.** Surface-Charge-Governed Ion Transport in Nanofluidic Channels. *Phys. Rev. Lett.,* 2004, vol. 93, 035901
- **DEKKER, C.** Solid-state nanopores. *Nat. Nanotechnol.,* 2007, vol. 2, 209-215
- **ROBIN, P. et al.** Long-term memory and synapse-like dynamics in two-dimensional nanofluidic channels. *Science,* 2023, vol. 379, 161-167
- **TENG, Y. et al.** Bioinspired nervous signal transmission system based on two-dimensional laminar nanofluidics: From electronics to ionics. *Proc. Natl. Acad. Sci.,* 2020, vol. 117, 16743-16748
- **XUE, L. et al.** Solid-state nanopore sensors. *Nat. Rev. Mater.,* 2020, vol. 5, 931-951
- **ACAR, E. T. ; BUCHSBAUM, S. F. ; COMBS, C. ; FORNASIERO, F. ; SIWY, Z. S.** Biomimetic potassium-selective nanopores. *Sci. Adv.,* 2019, vol. 5, eaav2568
- **FANG, A. ; KROENLEIN, K. ; RICCARDI, D. ; SMOLYANITSKY, A.** Highly mechanosensitive ion channels from graphene-embedded crown ethers. *Nat. Mater.,* 2019, vol. 18, 76-81
- **BOCQUET, L.** Nanofluidics coming of age. *Nat. Mater.,* 2020, vol. 19, 254-256
- **CRESSIOT, B. ; GREIVE, S. J. ; MOJTABAVI, M. ; ANTSON, A. A. ; WANUNU, M.** Thermostable virus portal proteins as reprogrammable adapters for solid-state nanopore sensors. *Nat. Commun.,* 2018, vol. 9, 4652
- **HALL, A. R. et al.** Hybrid pore formation by directed insertion of $\alpha$-haemolysin into solid-state nanopores. *Nat. Nanotechnol.,* 2010, vol. 5, 874-877
- **HE, Y. ; TSUTSUI, M. ; ZHOU, Y. ; MIAO, X.-S.** Solid-state nanopore systems: from materials to applications. *NPG Asia Mater,* 2021, vol. 13, 1-26
- **LIU, H. ; ZHOU, Q. ; WANG, W. ; FANG, F. ; ZHANG, J.** Solid-State Nanopore Array: Manufacturing and Applications. *Small,* 2023, vol. 19, 2205680
- **GADALETA, A. et al.** Sub-additive ionic transport across arrays of solid-state nanopores. *Phys. Fluids,* 2014, vol. 26, 012005

- **MA, L. et al.** Modulation of Ionic Current Rectification in Ultrashort Conical Nanopores. *Anal. Chem.,* 2020, vol. 92, 16188-16196
- **MACHA, M. ; MARION, S. ; NANDIGANA, V. V. R. ; RADENOVIC, A.** 2D materials as an emerging platform for nanopore-based power generation. *Nat. Rev. Mater.,* 2019, vol. 4, 588-605
- **KATIYAR, N. K. ; GOEL, G. ; HAWI, S. ; GOEL, S.** Nature-inspired materials: Emerging trends and prospects. *NPG Asia Mater,* 2021, vol. 13, 1-16
- **BLACK, D. M.** Cave Pearls in Carlsbad Caverns. *Sci. Mon.,* 1952, vol. 74, 206-210
- **SHAW, R. S. ; PACKARD, N. ; SCHRÖTER, M. ; SWINNEY, H. L.** Geometry-induced asymmetric diffusion. *Proc. Natl. Acad. Sci.,* 2007, vol. 104, 9580-9584
- **ZHANG, Z. et al.** Ultrathin and Ion-Selective Janus Membranes for High-Performance Osmotic Energy Conversion. *J. Am. Chem. Soc.,* 2017, vol. 139, 8905-8914
- **HSU, J.-P. et al.** Unraveling the Anomalous Surface-Charge-Dependent Osmotic Power Using a Single Funnel-Shaped Nanochannel. *ACS Nano,* 2019, vol. 13, 13374-13381
- **LIU, X. ; SKANATA, M. M. ; STEIN, D.** Entropic cages for trapping DNA near a nanopore. *Nat. Commun.,* 2015, vol. 6, 6222
- **FREEDMAN, K. J. et al.** Nanopore sensing at ultra-low concentrations using single-molecule dielectrophoretic trapping. *Nat. Commun.,* 2016, vol. 7, 10217
- **BELL, N. A. W. ; MUTHUKUMAR, M. ; KEYSER, U. F.** Translocation frequency of double-stranded DNA through a solid-state nanopore. *Phys. Rev. E,* 2016, vol. 93, 022401
- **LUO, L. ; HOLDEN, D. A. ; WHITE, H. S.** Negative Differential Electrolyte Resistance in a Solid-State Nanopore Resulting from Electroosmotic Flow Bistability. *ACS Nano,* 2014, vol. 8, 3023-3030
- **ZHANG, Z. ; WEN, L. ; JIANG, L.** Nanofluidics for osmotic energy conversion. *Nat. Rev. Mater.,* 2021, vol. 6, 622-639
- **FENG, J. et al.** Single-layer MoS2 nanopores as nanopower generators. *Nature,* 2016, vol. 536, 197-200
- **SIRIA, A. et al.** Giant osmotic energy conversion measured in a single transmembrane boron nitride nanotube. *Nature,* 2013, vol. 494, 455-458

- **YAZDA, K. et al.** High Osmotic Power Generation via Nanopore Arrays in Hybrid Hexagonal Boron Nitride/Silicon Nitride Membranes. *Nano Lett.,* 2021, vol. 21, 4152-4159
- **TSUTSUI, M. ; YOKOTA, K. ; LEONG, I. W. ; HE, Y. ; KAWAI, T.** Sparse multi-nanopore osmotic power generators. *Cell Rep. Phys. Sci.,* 2022, vol. 3, 101065
- **THAKUR, M. et al.** Wafer-Scale Fabrication of Nanopore Devices for Single-Molecule DNA Biosensing using MoS2. *Small Methods,* 2020, vol. 4, 2000072
- **HAUSMANN, D. M. ; KIM, E. ; BECKER, J. ; GORDON, R. G.** Atomic Layer Deposition of Hafnium and Zirconium Oxides Using Metal Amide Precursors. *Chem. Mater,* 2002, vol. 14, 4350-4358